# EUROPEAN PATENT SPECIFICATION

(11) **EP 1 557 462 B1**
(45) Date of publication and mention of the grant of the patent: **26.03.2008**
(21) Application number: 05009317.8
(22) Date of filing: 11.07.2003
(51) Int. Cl.: C12N 5/08, A61K 35/14, C07K 16/28, A61P 37/06

(54) **Transplant acceptance inducing cells of monocytic origin and their preparation and use**
Transplant-Akzeptanz induzierende Zellen monocytären ursprungs, sowie deren Herstellung und Verwendung
Cellules d'induction de l'acceptance d'un transplant d'origine monocytique et leur préparation et utilisation

(30) Priority: 12.07.2002 DE 10231655
(43) Date of publication of application: 27.07.2005
(62) Divisional of application: 03763823.6
(73) Proprietor: Blasticon Biotechnologische Forschung GmbH, 24063 Kiel (DE)
(72) Inventor: Kremer, Bernd Karl Friedrich, 24107 Kiel (DE); Fändrich, Fred, 24105 Kiel (DE); Schulze, Maren, 24238 Wittenberger Passau (DE)
(74) Representative: UEXKÜLL & STOLBERG

(56) References cited:
- WO-A-02/40646
- WO-A-98/53048
- WO-A-03/083092
- VOLPI I ET AL: "Postgrafting administration of granulocyte colony-stimulating factor impairs functional immune recovery in recipients of human leukocyte antigen haplotype-mismatched hematopoietic transplants." BLOOD. UNITED STATES 15 APR 2001, vol. 97, no. 8, 15 April 2001 (2001-04-15), pages 2514-2521, XP002227066 ISSN: 0006-4971
- SLOAND E M ET AL: "Pharmacologic doses of granulocyte colony-stimulating factor affect cytokine production by lymphocytes in vitro and in vivo." BLOOD. UNITED STATES 1 APR 2000, vol. 95, no. 7, 1 April 2000 (2000-04-01), pages 2269-2274, XP002227067 ISSN: 0006-4971
- WEISSMAN I L: "TRANSLATING STEM AND PROGENITOR CELL BIOLOGY TO THE CLINIC: BARRIERS AND OPPORTUNITIES" SCIENCE, AMERICAN ASSOCIATION FOR THE ADVANCEMENT OF SCIENCE,, US, vol. 287, no. 5457, 25 February 2000 (2000-02-25), pages 1442-1446, XP000919228 ISSN: 0036-8075
- LUCAS TREVOR ET AL: "Self-renewal, maturation, and differentiation of the rat myelomonocytic hematopoietic stem cell." FASEB JOURNAL, vol. 13, no. 2, February 1999 (1999-02), pages 263-272, XP002216397 ISSN: 0892-6638
- NAWA Y ET AL: "G-CSF reduces IFN-gamma and IL-4 production by T cells after allogeneic stimulation by indirectly modulating monocyte function." BONE MARROW TRANSPLANTATION, vol. 25, no. 10, 2 May 2000 (2000-05-02), pages 1035-1040, XP008012471 ISSN: 0268-3369

## Description

The invention relates to transplant acceptance inducing cells of monocytic origin and their preparation as well as their use for generating transplant acceptance.

Preferably, the invention relates to transplant acceptance inducing cells (in the following also called TAIC) which are derived from human monocytes.

The invention moreover describes the monoclonal antibody GM-7, which specifically recognises transplant acceptance inducing cells according to the invention which are derived from man.

In addition, the invention describes the use of the antibody GM-7 for detecting and/or selecting transplant acceptance inducing cells.

The term "transplantation" is used in the field of immunology for the transfer of cells, tissue or organs from one body to another. The need for transplantation arises from the finding that numerous diseases can be cured by transferring (transplanting) healthy organs, tissue or cells from one individual who is healthy in this respect - the donor - to an individual suffering from the disease concerned - the recipient or host.

Depending on the relationship between the donor and the recipient, the following types of transplant can be distinguished:
1. Autologous transplants: These are tissues or cells which are transplanted within one and the same individual from one area of the body to another.
2. Isologous transplants: This involves the transfer between genetically identical individuals. Examples of these are inbred strains of rats or mice. For man, isologous (syngeneic) transplants can be considered between genetically identical (monocygotic) twins.
3. Allogeneic transplants: This term designates transplants between genetically different members of the same species such as, for example, from one human individual to another or, in the case of experimental animals, from one inbred strain to another.
4. Xenogeneic transplants: This involves the transfer between different species such as, for example, the transfer of a monkey heart into a human being.

As a rule, autologous and isologous transplants cause no immunological problems in the sense of a rejection response. However, this is not true in the case of allogeneic and xenologous transplants. Such transplants are recognised as foreign by the recipient's immune system and rejected after a relatively short time. In the case of the allogeneic/ xenologous rejection response, T-lymphocytes (hereinafter called T-cells) play a central part. These cells recognise the foreign cells by way of their major histocompatibility complexes (MHC). The foreign MHC-complex sensitises the antigen-reactive T-cells of the recipient and, subsequently, the T-cells thus activated lead to the destruction of the donor cells or the donor tissue respectively. Cells with different MHC-complexes are also called "MHC-different".

Transplanting allogeneic or xenogeneic cells, tissues or organs necessarily leads to a situation where cells of two different individuals, i.e. of MHC-different cells, are present in the blood of the recipient at the same time. This phenomenon is referred to as "chimerism". A distinction is made bet tween micro-chimerism and macro-chimerism. Micro-chimerism refers to a state in which cells derived from the donor, e.g. after a blood or bone marrow transfusion or after transplanting lymphocyte-rich organs such as the small intestine or the liver, remain persistent in the MHC-different host but are detectable only in a few isolated cases. The term macro-chimerism is used when more than 5% of the cells detected in the recipient originate from the donor, a distinction needing to be made in both cases between the blood and the organ chimerism with the corresponding detection of donor cells in the recipient's blood or organs.

Chimerism can, to a certain extent, lead to tolerance vis-a-vis transplants. In the case of persons who, as a result of leukaemia or a lymphoma disease, for example, had to undergo an allogeneic bone marrow transplantation with a largely corresponding MHC pattern between the donor and the recipient, a "donor chimerism" was thus observed after corresponding myelo-ablative conditioning (destruction of the individual's own bone marrow and the blood cells derived therefrom) and subsequent stem cell transplantation. In the case of these patients, more than 99% of the cells detectable in the blood originated from the donor's stem cells and this chimerism formed the basis for the tolerance vis-à-vis all organs transplanted from this donor. This observation was confirmed in a series of clinical examples of subsequent kidney, liver and lung segment donations [Dey B., et al., "Outcomes of Recipients of both bone marrow and solid organ transplants: A review." Medicine (Baltimore) 77, 355-369 (1989)]. For a tolerance induction to occur, it is not decisive in this connection that a complete donor chimerism - defined as the presence of more than 90% donor cells in the peripheral blood of an allogeneic recipient - exists; instead, it has been found that a fraction of 5% donor cells - referred to as "mixed" chimerism - is sufficient to induce tolerance [compare survey by Acholonu I.N. and Ildstad S.T., "The role of bone marrow transplantation and tolerance: Organ-specific and cellular grafts", Curr. Opin. Organ. Transplant 4, 189-196 (1999)].

Although, basically, proof has thus been provided that the donor chimerism which accompanies a bone marrow transplantation (BMTx) induces tolerance, it has nevertheless not been possible so far to carry out a successful allogeneic BMTx in the case of completely MHC-different donor and recipient constellations. This is due to the fact that the preconditioning required for this, i.e. conditioning of the recipient (see above) holds three substantial types of risk for the recipient. Firstly, the bone marrow toxicity of the chemotherapeutic agents used, which are administered together with or without additional radiation treatment, leads to a significant morbidity; secondly, there is the risk of the transplanted bone marrow being rejected in spite of prior conditioning; and, finally, there is the risk of a graft versus host disease (GvHD) in the case of which the T-cells transferred into the host together with the transplant turn against the host's organism [compare Wolff S.N., "Hematopoietic cell transplant from volunteer unrelated or partially matched related donors: Recent developments." Curr. Opin. Organ. Transplant 5, 372-381 (2000)]. This disease occurs whenever the recipient's immune system has been weakened by the prior conditioning to such an extent that the T-cells transferred with the bone marrow are capable of causing a lethal rejection in the recipient.

To avoid the problems outlined above, efforts are under way at present to design the conditioning of the recipient to be as weak as possible. However, the greater the MHC difference between the donor and the recipient, the stronger a conditioning treatment must be chosen in order to prevent the rejection of the transplanted bone marrow; this again leads to an increase of the risk of a graft versus host disease (GvHD).

So far, no detailed investigation has been carried out regarding the time for which the induced chimerism must remain detectable in the recipient's blood in order to guarantee a stable tolerance situation for a donor organ. Reliable data from the mouse model, however, indicate that a macrochimerism detectable for two weeks is already sufficient for tolerance to arise [Weckerle, T. et al. "Allogeneic bone marrow transplantation with co-stimulatory blockade induces macro-chimerism and tolerance without cytoreductive host treatment." Nat. Med. 6, 464-469 (2000)].

The need to develop alternatives to the immunosuppressants available at present to generate tolerance arises from the angle of two major points of view. On the one hand, the immuno-suppressants clinically available so far have not been capable of preventing chronic transplant rejection and, on the other hand, taking immunosuppressants permanently is accompanied by considerable side effects which make the patient prone to viral, bacterial and mycotic infections and which generate substantial risks through the formation of malignoma and diseases of the cardiovascular system, in particular by myocardial infarction and by cardial insufficiency [see Wheeler, D. C. and Steiger, J. "Evolution and Etiology of Cardiovascular Diseases in Renal Transplant Recipients" Transplantation 70, 41-45 (2000)].

Munn et al. have described a macrophage-induced T-cell suppression, which is based on the selective elimination of Tryptophan and/or increase in one or more Tryptophan metabolites in the environment surrounding the cells [see Munn et al. "Inhibition of T Cell Proliferation by Macrophage Tryptophan Catabolism" J. Exp. Med. 189, 1363-1372 (1999)]. Starting from this observation, the authors suggest to modify T-cell mediated immuno-reactions by the modification of the local extracellular tryptophan concentration, in particular by inhibiting or increasing the IDO-mediated Tryptophan-metabolism, see for instance the US-patens 6,482,416 and 6,451,840. Thus, this approach only provides a further antigen independent and unspecific immunosuppression directed against T-cells.

What is urgently needed, however, is a clinically applicable concept for inducing donor-specific tolerance, i.e. a concept according to which the host's immune response vis-à-vis the tissue antigens present on the organ transferred from the donor is specifically suppressed but in the case of which the host's immunocompetence is otherwise completely preserved.

By way of such a concept, a substantial reduction in the discrepancy between organ demand and organ supply would arise in the medium term since, in this way, far fewer organs would be lost as a result of acute and chronic rejection processes. The lack of suitable organs can be explained by way of statistics from the USA dating from February 2001:

**Table 1: Up-to-date data from "UNOS (United Nations Organ Share Registry) National Patient Waiting List for Organ Transplant (February 2001)"**

| Type of transplant | Number of patients on the waiting list |
|---|---|
| Kidney | 47,996 |
| Liver | 17,151 |
| Pancreas | 1,060 |
| Pancreatic islet cell | 185 |
| Kidney-pancreas | 2,442 |
| Intestine | 151 |
| Heart | 4,222 |
| Heart-lung | 210 |
| Lung | 3,721 |
| Total number of patients | 74,800 |

This explains the immense national and international efforts being made to develop concepts for inducing donor-specific tolerance.

It is consequently the problem underlying the invention to provide means for influencing the T-cells of the recipient organism in such a way that they tolerate and/or accept the foreign cells, tissues or organs concerned in the long term without rejection. The constitution of these means and their preparation or use should not cause any ethical and/or legal problems and it must be possible to prepare them rapidly for the intended therapeutic use in the quantities required for the purpose and at acceptable manufacturing costs.

For the solution of the problem, the transplant acceptance inducing cells of monocytic origin (TAIC) according to the invention from vertebrates, in particular form mammals, and more preferred from humans are provided. It has surprisingly been found that monocytes from the blood of the donor (organ donor), modified according to the invention, are capable of protecting the recipient organism, in the case of preoperative and/or postoperative administration, against its own body T-cells activated by the foreign MHC-complex thereby preventing the rejection of the transplant. When used as "cellular therapeutic agents" for inducing transplant tolerance, the modified cells cause no or no noteworthy side effects in the sense of cellular rejection, induction of tumours, in particular of malignant tumours and graft versus host disease in the patient concerned.

It has surprisingly been found that the process according to the invention leads to the *in vitro* modification of monocytes in such a way that cells are obtained which, after injection into a not immune suppressed allogeneic recipient, are capable of preventing the naturally occurring immune response against cells or tissue of the donor and which are thus capable of circulating in the peripheral blood for at least three weeks. Following the administration of approximately 10⁵ cells / kg body weight (BW), the resulting chimerism of GM-7 positive cells, see below, is in the region of 5-20%. This transient chimerism induces
a) long-term acceptance for subsequent organ transplantations from the same donor, preferably within 10 days following the intravenous administration of the cells and
b) long-term acceptance in combination with short-term immunosuppressive treatment when the cells are used subsequent to organ transplantation.

As shown in Example 10, the immunosuppressive effect of the cells of the invention are not associated with the macrophage-induced T-cell suppression by expression of the tryptophan degrading enzyme Indolamine-2,3-Diogenase (IDO), as observed by Munn et al., (see above). Rather, the TAIC of the invention induce a specific tolerance versus the donor in the recipient, by inactivating alloreactive T-cells on the one hand and by inducing the formation of regulatory T-cells in the recipient on the other hand, see Examples 12 and 13.

Macrophages derived from monocytes, which inhibit the proliferation of T-cells by an IDO-mediated elimination of Tryptophan in the environment surrounding the cells, are therefore not included in the invention.

### Description of the Figures

- Figure 1:: Flow cytometric determination of the binding capacity of GM-7 to original monocytic cells before (graph on the left hand side) and after (graph on the right hand side) modification of the cells according to the invention. The x axis indicates the number of bound cells.
- Figure 2A:: Kaplan-Meier survival curves of heart transplants following heterotopic heart transplantation with and without preoperative administration of donor-derived TAIC in the rat model (n = 10 animals per group).
- Figure 2B:: Histological preparation of an LEW allogeneic transplant on POD (postoperative day) 150 following heterotopic transplantation into the abdomen of a DA recipient rat that had been pre-treated on day -7 with 10⁶ LEW-derived TAIC. Magnification factor: 40.
- Figure 2C:: Histological preparation from the thymus of a DA rat pre-treated with 10⁶ LEW-derived TAIC following labelling of the preparation with the mono-clonal antibody I1.69 specific for LEW-MHC class I. Magnification factor: 40.
- Figure 2D:: Flow cytometric detection of donor-derived cells in four DA recipient rats not subjected to immunosuppression following the injection of LEW-derived TAIC (rats 1-3: 10⁶ cells / kg × BW; rat 4: 10⁴ cells / kg × BW).
- Figure 3:: Kaplan-Meier survival curves of heart transplants following heterotopic heart transplantation with and without postoperative administration of donor-derived TAIC in combination with an initial administration of 4 cycles of Cyclosporin A (CSA; 5 mg / kg × BW) in the rat model (n = 6-10 animals per group).
- Figure 4A-C:: Kaplan-Meier survival curve of heart (A), liver (B) and kidney (C) transplants following heterotopic (A and C) or orthotopic (B) transplantation respectively with and without preoperative administration of donor-derived TAIC (n = 6 animals per group).
- Figure 4D-F:: Histological preparations of DA transplants of the kidney (D), the liver (E) and the skin (F) following heterotopic transplantation into a LEW recipient rat.
- Figure 5A:: Kaplan-Meier survival curves of heart transplants following heterotopic heart transplantations of different combinations of inbred strains with and without postoperative administration of donor-derived TAIC in combination with an initial administration of 4 cycles of CSA (5mg/kg × BW) in the rat model (n = 6 animals per group).
- Figure 5B:: Kaplan-Meier survival curves of heart transplants following heterotopic heart transplantations 7 days after the administration of differently treated blood monocytes from LEW donor animals (n = 6 animals per group).
- Figure 6A:: Kaplan-Meier survival curves of lung transplants following orthotopic left sided lung transplantations in pigs ("outbred minipigs") using a triple immunosuppression of CSA, Azathioprin (AZA) and steroids (STE). (n = 4 animals per group).
- Figure 6B:: X-ray picture of the thorax (posterior-anterior technique) of two single-side left lung transplanted pigs ("outbred minipigs") on POD (postoperative day) 41 and 55. The triple immunosuppression of CSA, AZA and steroids was discontinued on day 28 after transplantation.
- Figure 7:: Mixed lymphocytes culture of CD14⁺ monocytes from an individual B (GM-7⁻: grey column; GM-7⁺: black column), responder cells from an MHC-discordant donor A and irradiated cells from a donor B to compare the suppressor activity of CD14⁺/GM-7⁺ and CD14⁺/GM-7⁻ cells.
- Figure 8:: Mixed lymphocyte culture of PHA-stimulated lymphocytes (PhaLy) and TAIC ("Mo+Ly" or "Mo"), preincubated in two experiments with an inhibitor (1-MT) of indolamine-2,3-dioxygenase (IDO) for determining the influence of 1-MT on the suppressor activity of the TAIC.
- Figure 9:: Flow cytometric determination of the amount of CD14⁺-monocytes and of CD2⁺-lymphocytes in the monocyte fraction as well as the amount of the CD-14⁺/CD3⁺-cells effective as TAIC, for determining the influence of purifying the cells for enriching monocytes at the beginning of the culture on the formation of immunosuppressive CD14⁺/CD3⁺-cells.
- Figure 10:: Flow cytometric determination of GM-7-expression in the blood of patients postoperatively prior (left Figure) and after (right Figure) injection of TAIC, for determining the influence of TAIC on the *in vivo*-GM-7-expression in blood cells.

### Summary of the invention

The main steps of the process according to the invention for the preparation of TAIC of monocytic origin comprise:
(a) Isolating monocytes from blood, preferably from human blood;
(b) Propagating the monocytes in a suitable culture medium which contains the macrophage colony stimulating factor (hereinafter referred to as M-CSF) as growth promoting agent;
(c) Stimulating the monocytes with γ-interferon (hereinafter called γ-IFN); and
(d) Obtaining the transplant acceptance inducing cells formed in stage c) by separating the cells from the culture medium.

According to the invention, it has been shown that the stimulation with γ-IFN represents a decisive step in the preparation of TAIC (see Example 7).

With respect to the present invention' the term "TAIC" (transplant acceptance inducing cells of monocytic origin) designates the cell population, which is obtained from step (d) of the process described above. This cell population comprises next to the cells derived from monocytes, which are effective according to the invention, also lymphocytes, see Example 11, as well as optionally further cells derived from the buffy-coat, as for instance granulocytes. The amount of cells derived from monocytes within the TAIC-population is preferably 50 to 90 %, more preferably 60 to 70 %, referring to the total cell number.

With respect to the present invention the term "total cell number" refers to the amount of vital cells in the cell population under consideration. This amount can be determined by the "trypan blue dye exclusion technique", since this dye allows to distinguish vital cells from non-vital cells by optical means.

The TAIC according to the invention may usually be used in a quantity of 10⁴-10⁶ cells per kilogram body weight, preferably 10⁵ cells per kilogram body weight, to induce transplant acceptance. TAIC administration may be carried out repeatedly, in the case of a large MHC difference preferably three times at intervals of approximately 10 days where the administration of the cells may take place prior to or after transplantation (see below). The total number of cells necessary for this purpose can be provided within 6 to 8 days after the blood was taken.

The cells according to the invention (TAIC) have proved to be risk-free regarding the formation of malignoma, both in the animal test and in culture; this is a result which could not have been expected in any other way because of the nature of the original monocytic cell from which the cells according to the invention are derived.

As explained below, further sub-populations of cells having optimized effectivity according to the invention, can be isolated from the fraction of cells present in TAIC, which are derived from monocytes.

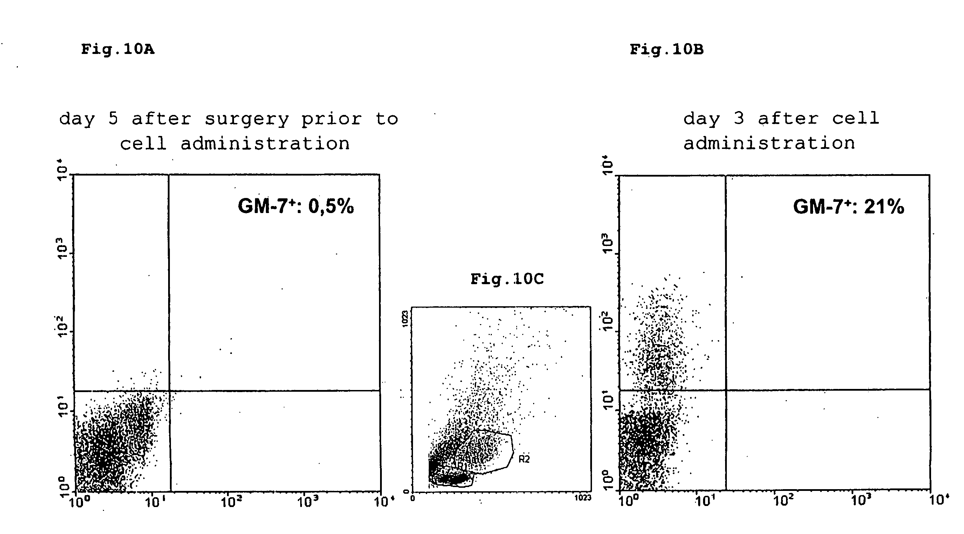

Following *in vitro* cultivation and stimulation of the original cells (monocytes) with γ-interferon TAIC are formed, which comprise a sub-population of cells, see Example 9, which binds the monoclonal antibody GM-7, which is expressed by the hybridoma cell line DSM ACC2542. The monoclonal antibody GM-7 is an antibody of the immunoglobulin isotype IgG₂ₐ, the light chain of which exhibits the kappa-isotype. The characteristic property of this antibody is its stringent capacity to bind to the monocytes modified by the culture conditions according to the invention since original monocytic cells are not recognised, i.e. binding of the antibody to the original cells does not take place, (see Example 9). In addition, it was demonstrated with 20 volunteers that GM-7 does not bind to human cells in the peripheral blood, see Figure 10.

The antibody was prepared by immunising mice with TAIC derived from human monocytes using methods known to the person skilled in the art (Davis, W.C. "Methods in Molecular Biology: Monoclonal Antibody Protocols", New York: Humana Press Inc. Totowa, 1995). A hybridoma cell line was then produced by fusion of a B cell generating the antibody and a myeloma cell from the mouse. Methods used for the preparation of such cell lines are known in the state of the art (Davis, W.C. "Methods in Molecular Biology: Monoclonal Antibody Protocols", New York: Humana Press Inc. Totowa, 1995; Kohler, G., Milstein, C. "Continuous cultures of fused cells secreting antibody of predefined specificity", Nature 256, 495-497 (1975)). The hybridoma cell line producing the antibody GM-7 was deposited according to the rules of the Budapest Convention at DSMZ (Deutsche Sammlung von Mikroorganismen und Zellkultur GmbH, Braunschweig, Germany) under the accession no. DSM ACC2542 (date of deposit: May 13, 2002).

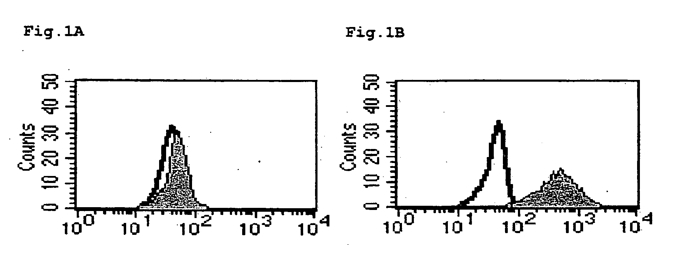

Figure 1 shows the binding capacity, determined by flow cytometry, of GM-7 to monocytic cells after *in vitro* modification according to the invention. It can be seen that the CD14-positive monocytes obtained directly from buffy-coat do not bind the antibody GM-7 (the cloud shaded grey is congruent with the non-shaded antibody control). In contrast, following cultivation in the presence of M-CSF and stimulation with γ-IFN, part of the monocytes express an antigen which is recognised by the monoclonal antibody GM-7. The monoclonal antibody GM-7 was characterised as isotype κ-IgG₂ₐ. The process according to the invention consequently leads to a change in the phenotypic pattern of the antigen expression on the cell membrane of the modified monocytes (Figure 1).

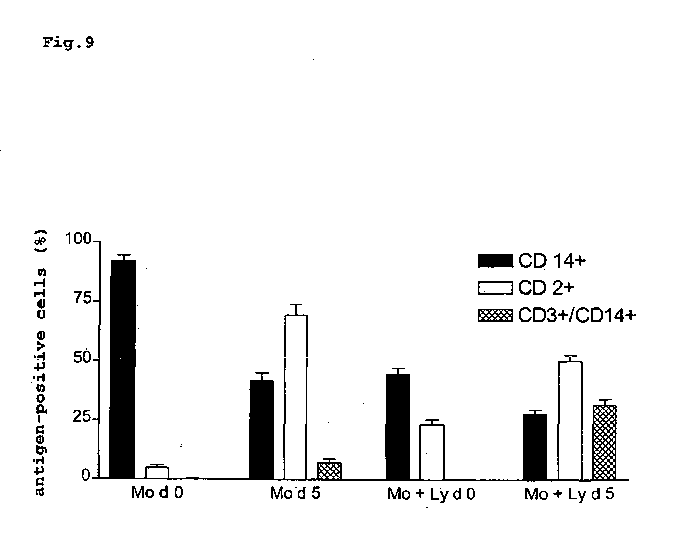

The monoclonal antibody GM-7 binds specifically to that cell population which, among those cells produced by the process according to the invention, induce the most effective transplant acceptance (see Figure 9)

Therefore, the invention describes such TAIC, which are capable of binding the antibody GM-7. These cells are subsequently designated as TAIC_{Gm7}.

The antibody GM-7 therefore represents an extraordinarily effective and easy to handle agent for selecting and purifying the cells inducing transplant acceptance (TAIC). By means of the antibody, it is possible to generate a homogeneous and highly effective TAIC population.

The transplant acceptance inducing cells formed in step c) of the above described process of the invention, which express the antigen binding to the antibody GM-7, may either be selected directly from the culture medium after step c), or they may be selected from the cell population obtained after separating the cells from the culture medium according to step d) of the above-mentioned process of the invention by binding to the antibody GM-7 produced by the hybridoma cell line DSM ACC2542.

To select the TAIC, the antibody is contacted with the sample under conditions which permit binding of the antibody to the transplant acceptance inducing cells present in the sample. The reaction complexes resulting from the binding reaction are subsequently separated from the sample. For this purpose, the antibody can be immobilised on a carrier material before contact with the sample; for example, it can be bound to a matrix suitable for chromatographic purposes or to so-called "magnetic beads". This procedure allows to select and concentrate transplant acceptance inducing cells from large volumes of sample.

To obtain the transplant acceptance inducing cells, the bond between the antibody and the transplant acceptance inducing cells is separated after the isolation of the reaction complex from the sample. This can be effected by methods known in the state of the art such as e.g. by competitive displacement or by washing with salt solutions. Corresponding methods are for instance described by Utz U. et al. ("Analysis of the T-cell Receptor repertoire of human T-cell leukemia virus type-1 (HTLV-1) Tax-specific CD8+ Cytotoxic T Lymphocytes from patients with HTLV-1 associated disease: Evidence for the oligoclonal expansion" J. of Virology Feb. 1996, 843-851).

Moreover, the monoclonal antibody GM-7 permits the qualitative and quantitative detection of the transplant acceptance inducing cells of monocytic origin in blood and/or tissue samples of the patient *in vitro*. This patient may, for example, be the recipient of an organ yet to be transplanted or already transplanted. The formation of reaction complexes in the sample which indicate the presence and, if applicable, the quantity of the transplant acceptance inducing cells is detected by known methods.

To detect the reaction complexes, it is possible in this case to couple ("label") the antibody GM-7, for example, directly with a detectable molecule which is e.g. covalently bound to the antibody. Suitable detectable molecules are described in large numbers in the field of molecular diagnostics and include, among others, fluorescent dyes such as fluorescein isothiocyanate or tetramethyl rhodamine-5-isothiocyanate, luminescent dyes, radioactively labelled molecules and enzymes such as peroxidases (compare Lottspeich, F., Zorbas, H. "Bioanalytik", Spektrum Akademischer Verlag GmbH, Heidelberg-Berlin, 1998)

The detection of the antibody takes place dependent of the molecule selected for labelling of the former. In connection with the present invention, the antibody GM-7 was coupled with the fluorescent molecule fluorescein isothiocyanate (FITC) so that the detection of the antibody could be carried out by means of flow cytometry and/or fluorescence microscopy. Methods for labelling antibodies with FITC are known to the person skilled in the art who operates in the field.

Alternatively, the reaction complex can also be detected in a two-stage process using secondary antibodies. In this connection, the unlabelled antibody GM-7 can be detected in the reaction complex with a further labelled antibody (compare Lottspeich, F., Zorbas, H. "Bioanalytik", Spektrum Akademischer Verlag GmbH, Heidelberg-Berlin, 1998). This two-stage method of detection is considerably more sensitive than the direct detection of binding of the antibody since several labelled secondary antibodies can bind to one GM-7 antibody (signal amplification).

The antibody GM-7 consequently allows the detection of TAIC in the peripheral blood of the patient treated with TAIC, for example in the form of "monitoring", during which the number of cells in the peripheral blood is determined at specific time intervals. According to the invention, it has been found that the presence of TAIC prepared from monocytes of the donor correlates, in the animal test in the peripheral blood of the transplant recipient, with the tolerance of the transplanted organ. This finding consequently permits the clinician to wean off or to reduce the dosage of the immuno-suppressants that are optionally administered stepwise. Previously, it had not been possible from the clinical point of view to provide accurate evidence as to whether the immune system of a patient exhibited tolerance at a given point in time following transplantation.

As is obvious to the person skilled in the art, it is possible to prepare monoclonal antibodies against TAIC from monocytes also of non-human vertebrates, in particular from monocytes of primates and pigs modified according to the invention. In this respect, the immunisation of the corresponding host animals and the generation of the corresponding hybridoma cell line are carried out as described above for the TAIC of human origin. The generation of effective TAIC populations from other species provides an important contribution in the field of xenogeneic transplantation medicine.

The invention also discloses a sub-population of the TAIC, which co-express the antigens CD3 and CD14 on their cell surface. These cells are subsequently indicated as TAIC_{CD3+/CD14+}. Such cells have to date not been reported in the state of the art. Monocytes and known cells derived from monocytes do carry the surface marker CD14, however, they do not additionally carry the surface marker CD3 at the same time.

TAIC which co-express the surface antigens CD3 and CD14 may either be directly selected from the transplant acceptance inducing cells formed in step c) of the above described process of the invention, or they may be selected from the cell population obtained after separating the cells from the culture medium according to step d) of the above-mentioned process of the invention, or they may alternatively be selected from the TAIC_{GM7} population.

Further, it has been shown that the TAIC_{CD3+/CD14+} express the genes Foxp3, CTLA4 and Integrin α_{E}β₇ strongly (see Example 12). In contrast, these genes are not or only to a small extent expressed by the original monocytes. The upregulation of the expression of the genes Foxp3, CTLA4 and Integrin α_{E}β₇ is therefore a characteristic of TAIC_{CD3+/CD14+}cells.

As discussed in Example 12, the expression of the markers FoxP3, CTLA4 and Integrin α_{E}β₇ was previously only described for regulatory T-lymphocytes. T-lymphocytes, which co-express the surface antigens CD4 and CD25 are a sub-population of regulatory T-lymphocytes, which are also indicated as "suppressor cells". It is their function to suppress the immune response of the body. In particular, Foxp3 is seen as a specific transcription factor, which serves as a control gene for the development of regulatory T-cells, and which is specifically expressed by these cells. The TAIC_{cD3+/CD14+}-cells express at least 1 × 10⁻⁹, more preferably at least 5 × 10⁻⁹, and in particularly preferred manner at least 1 × 10⁻⁸ µg Foxp3-RNA per µg total RNA

CTLA4 is similarly viewed as a marker for the detection of the regulatory function of T-lymphocytes, in particular of CD4/CD25 positive T-lymphocytes (see the literature cited in Example 12). The TAIC_{CD3+/CD14+}-cells should preferably express at least 5 × 10⁻⁷, more preferably at least 3 × 10⁻⁶ and in a particularly preferred manner at least 5 × 10⁻⁶ µg CTLA4-RNA per µg total RNA.

Integrin α_{E}β₇, which recognizes epithelial Cadherin was recently described by Lehmann et al. in PNAS 99, pages 13031-13036 (2002) as a new marker for a sub-population of highly potent regulatory T-lymphocytes, which interact with the epithelial environment. The expression of the Integrin α_{E}β₇-RNA should according to the invention amount in TAIC_{CD3+/CD14+}-cells to preferably at least 1 × 10⁻¹², more preferably to at least 1 × 10⁻¹¹, and in a particularly preferred manner to at least 1 × 10⁻¹⁰, and most preferably to at least 1 × 10⁻⁹ µg per 1 µg total RNA.

As shown in the Table in Example 12, the direct co-culturing of the TAIC of the invention with lymphocytes leads to a significant increase in the number of regulatory T-lymphocytes, in particular of CD4/CD25 double-positive cells in the lymphocyte population with strongly up-regulated expression of the genes Foxp3, CTLA4 and Integrin α_{E}β₇. The Example further demonstrates that this effect is not observed if TAIC are indirectly co-cultured with lymphocytes.

These results indicate that stimulation of the formation and/or propagation of regulatory T-lymphocytes by TAIC is involved in the induction of transplant acceptance by the TAIC of the invention.

Example 13 confirms this hypothesis. In this Example, lymphocytes from the recipient animals of Examples 3, 4, 5, 6 and 7 were incubated with TAIC from the respective donor animals *in vitro*. For the induction of tolerance, TAIC pre-incubated with the lymphocytes from the recipients were injected to the animals instead of TAIC. Donor specific tolerance could be induced also in this manner, while animals, to which recipient lymphocytes not co-cultivated with donor derived TAIC were administered, did not develop tolerance.

The TAIC of the invention can be used as such as pharmaceutical preparation. The cells obtained from step d) of the method of the invention as described above can be used directly. About 10 - 50 % of the total cells of the populations so obtained is formed by lymphocytes and granulocytes, which stem from the initial monocyte isolate (buffy-coat). These cells support the formation of the TAIC of the invention derived from the monocytes in the culturing step (see Example 11); they do not interfere with the tolerance induction if the TAIC of the invention are used as a pharmaceutical preparation.

However, the sub-populations TAIC_{GM7} and/or TAIC_{CD3+/CD14+} may be isolated from the totality of the TAIC population obtained from process of the invention (see above) and may be used for tolerance induction.

In culture media (see Example 2), the TAIC or the TAIC_{GM7} and/or TAIC_{CD3+/CD14+} may be kept for at least 48 hours without their tolerance inducing effect becoming lost.

For use as pharmaceutical preparation, the TAIC or the sub-populations TAIC_{GM-7} and/or TAIC_{CD3+/CD14+} suspended in e.g. human AB serum (universally suitable for use) can be administered intravenously as short transfusion. For the induction of transplant acceptance in the case of allogeneic transplantation, the TAIC generated from the monocytes of the donor or the sub-populations TAIC_{GM7} and/or TAIC_{CD3+/CD14+} can be injected into the MHC-different recipient either pre-operatively or postoperatively. In the case of preoperative administration, the TAIC should be injected once to three times approximately 1 week before the operation. In the case of postoperative administration, the period between the operation and the single administration of the cells should not be longer than 7 days. The TAIC according to the invention or the sub-populations TAIC_{GM7} and/or TAIC_{CD3+/CD14+} are then capable of repelling the T-cell response of the recipient's immune system against the transplant and to persist in the recipient blood for a sufficiently long period of time to guarantee long-term transplant acceptance.

Preoperative intravenous injection can be considered in connection with donations from living individuals; however, if a corpse donation (blood and organ from a dead body) is at issue, postoperative administration of the TAIC according to the invention or the sub-populations TAIC_{GM7} and/or TAIC_{CD3+/CD14+} may be preferred. In the case of a corpse donation, the body of the donor is flushed with a perfusion medium by canalisation of the principal artery for purposes of organ preservation. In this case, the venous blood is normally sucked out via the vena cava and discarded. To obtain the TAIC to be used according to the invention, the venous blood can be collected and processed as described in Example 1. In the alternative, in the case of corpse donation, TAIC may also be obtained from cells (lymphocytes and monocytes) from the donor spleen.

In the case of postoperative application of the TAIC according to the invention in connection with corpse donation, the interval between the transplantation and the application of the cells can be overcome by combination with immuno-suppressants, in order to prevent an acute rejection of the organ in the interval between the transplantation and the provision of the TAIC obtained from the donor blood. In this connection, therapeutic combinations with conventional immuno-suppressants such as, for example, calcineurin inhibitors such as cyclosporin A (CSA) or tacrolimus or with azathioprine (AZA), mycophenolate mofetil, rapamycin, monoclonal antibodies (ATG, ALG, but neither with Dicliziumab or Basiliximab) or steroids (STE) can be considered. The presence of immuno-suppressants (except for known IL-2-receptor-α monoclonal antibodies such as Dicliziumab and Basiliximab) in the recipient blood has no negative effect on the effectiveness of the immuno-suppressants, on the one hand, or the TAIC, on the other hand.

In this sense, the cells of monocytic origin which have been modified according to the invention can be used as "vehicle for tolerance transfer" for any cellular transplant (such as islet cells, hepatocytes, adult stem cells and for any other programmed cell type or tissue type) and organ (such as e.g. kidney, liver, heart) insofar as they are genetically identical to the cells to be transplanted (organs), i.e. they must originate from the donor him/herself or from identical twins thereof. The TAIC fulfil their protective function by allowing the transplanted cells / organs to become adherent in the new environment, thus saving the recipient from suffering the side effects of a longterm immunosuppressive therapy.

### Detailed description of the invention

The starting cells for the process according to the invention are blood monocytes. These are preferably monocytes from human blood. For purposes of inducing transplant acceptance, the cells must originate from the donor of the transplant (or his/her identical twin). In the case of xenogeneic transplantations of e.g. monkey or pig organs into man, the TAIC according to the invention must consequently be derived from the monocytes of the donor animal concerned.

To obtain the monocytes, the blood can first be separated, after the usual treatment with an anticoagulant, into plasma and into white and red blood cells using methods known in the art, preferably by centrifugation. After centrifugation, the plasma will be present in the supernatant; below it, there is a layer which contains the white blood cells in their entirety. This layer is also referred to as buffy-coat. Below this is the phase containing the red blood cells (hematocrit).

In connection with the process according to the invention, the buffy-coat layer is first isolated and separated to obtain the monocytes e.g. by centrifuging according to known methods. According to a preferred process embodiment, the buffy-coat layer is applied onto a lymphocyte separation medium (Ficoll-Hypaque) and centrifuged (see Example 1). Example 1 describes the preferred embodiment of the invention, wherein the erythrocytes and dead cells still contained in the buffy-coat are separated by centrifuging, and the white blood cells including the monocytes are present as an isolate on the separation medium. Thereafter, the white phase of monocytes may be carefully pipetted off, and, for enrichment of the monocytes within the isolate, are repeatedly centrifuged and washed. In the course of this process, the monocytes will assemble at the bottom of the centrifuge vessel together with a part of the lymphocytes.

According to a particularly preferred embodiment of the method of the invention, the conditions for obtaining the monocyte containing isolate are controlled such that the isolate contains about 10 - 50 % lymphocytes next to the monocytes, by reference to the total number of cells. Preferably, the isolate contains about 50 - 90 %, in a particularly preferred manner 60 - 70 % monocytes and about 10 - 50 %, in a particularly preferred manner 20 - 50 % lymphocytes, each by reference to the total cell count, wherein the difference will optionally be provided by granolocytes.

As shown in Example 11, the presence of lymphocytes in a magnitude of 20 - 30 %, referred to the total cell count, during the culturing of the original monocytes with MCSF and γ-interferon will lead to the generation of a significantly higher amount of CD3/CD14-double positive TAIC, as will be the case if only a few lymphocytes (about 5 %) are present.

To prepare a sufficient quantity of TAIC, it is first necessary to allow propagation of the monocytes. For this purpose, known growth media suitable for monocytes can be used; however, the medium must contain the growth factor M-CSF (macrophage-colony-stimulating-factor). M-CSF (also-called CSF-1) is produced by monocytes, fibroblasts, lymphocytes and endothelial cells. The concentration of M-CSF in the culture medium may preferably amount from 2 to 20 µg/l medium, more preferably 4 to 6 µg/l and in particularly preferred manner 5 µg/l.

Subsequently or simultaneously, the cells must be stimulated with γ-IFN, i.e. cultured in the presence of γ-IFN. The stimulation of the monocytes with γ-IFN takes place after an initrial propagation phase lasting 3 to 6 days in the culture medium containing the growth factor. Preferably on day 4 after the beginning of cultivation, γ-IFN stimulation is carried out and this stimulation is extended over a period of preferably 24 to 72 hours, more preferably 48 hours under incubator conditions i.e. at 37°C and in a 5% CO₂ atmosphere.

The concentration of γ-IFN in the medium may be 0.1 to 20 ng/ml, preferably 1 to 10 ng/ml and particularly preferably 5 ng/ml.

The stimulation with γ-IFN may begin simultaneously with the propagation of the monocytes in the medium containing the growth factor. However, stimulation after a 3 to 6 day long initial propagation phase, as indicated above, is preferred. The propagation of the cells and stimulation with γ-IFN should, overall, preferably not take more than 8 days. In any case, treatment with γ-IFN should be carried out such that after the propagation phase it lasts for at least 24 hours, at maximum 72 hours, preferably 48 hours. The period for propagation and stimulation of the cells should consequently last for a total of preferably 4 to 8 days.

According to a preferred embodiment of the invention, the propagation and stimulation with γ-interferon is carried out as indicated in Example 2 in such a way that the monocytes are first propagated in a culture medium containing the growth factor and that γ-IFN is added to the culture medium after 3 to 6 days in such an amount that a concentration of 0.1 to 20 ng/ml, preferably 1 to 10 ng/ml and particularly preferably 5 ng/ml is obtained in the medium.

Preferably, the process according to the invention is carried out in culture vessels the surface of which has previously been coated with fetal calf serum (FCS) or alternatively with human AB serum (see Example 2). Coating with FCS can take place by covering the surface of the culture vessels with FCS before use and, following a period of interaction of a few hours, in particular 4 to 72 hours, preferably 12-48 hours and in particular 24 hours, removing the FCS not adhering to the surface in a suitable manner.

During the culturing step the cells will settle at the bottom of the culturing vessel after about 24 hours. Due to their adhesive properties the monocytes and the TAIC, derived from the monocytes during the process, will then adhere to the bottom of the respective culture vessel. If, as described in Example 2, the culture medium is changed during cultivation, the supernatant is initially carefully removed, for instance by pipetting off or decanting, and subsequently fresh culture medium is filled in. Preferably however, the cells adhering to the bottom are not or only carefully washed, so that any lymphocytes present are not removed.

Removing the adhering cells can take place mechanically, e.g. by means of a fine cell scraper or spatula.

According to a preferred embodiment of the process according to the invention, however, the complete removal of the cell takes place by treatment with a suitable enzyme, e.g. with trypsin (see Example 2). The trypsin solution (0.1 to 0.025 g/l, preferably 0,05 g/l) can be allowed to act onto the cells for 2 to 10 minutes at 35°C to 39°C, preferably at 37°C, in the presence of 5% CO₂.

The enzyme activity is then blocked in the usual manner and the now freely floating TAIC can be obtained in the usual way by centrifuging. They are then available for immediate use, optionally in suspension in a suitable medium, e.g. in PBS. However, they can also be kept for several days, in particular for approximately 2 to 3 days, in a nutrient medium (see Example 2); wherein this preservation medium should contain neither the growth factor nor γ-IFN. The cells can be kept in such a nutrient medium as TAIC for at least 48 hours.

For storage over longer periods, the cells can be deep frozen. Protocols for deep freezing of living cells are known in the state of the art, compare Griffith M. et al., ("Epithelial Cell Culture, Cornea", in Methods of tissue engineering, Atala A. and Lanza R.P., Academic Press 2002, chapter 4, pages 131-140). A preferred suspension medium for deep freezing of the cells according to the invention is FCS containing DMSO.

The cell suspension containing transplant acceptance inducing cells obtained from steps c) or d) may be further purified with respect to those cells, which bind the antibody GM-7, so as to obtain a sub-population TAIC_{GM7}. Methods for such purification are described above in detail.

Such cells are selected from the TAIC population, which co-express the antigens CD3 and CD14 on their cell surfaces. Methods for selecting such cells are known in the art. Examples for such methods are the "Fluorescent-Activating Cell Sorting" (FACS), the "Immuno Magnetic Bead Sorting" and the "Magnetic Activated Cell Sorting" (MACS), or the so-called "Rosetting Method" [see Gmelig-Meyling F. et al. "Simplified procedure for the separation of human T- and non-T-cells", Vox Sang. 33, 5-8 (1977)].

The selection of the TAIC sub-population TAIC_{CD3+/CD14+} can take place directly from the TAIC population obtained from steps c) or d) of the above-described process of the invention, or from the TAIC_{GM7}-sub-population. The latter way to proceed means that a stepwise enrichment of TAIC_{CD3+/CD14+} will take place.

In a preferred embodiment of the invention the TAIC according to the invention are used per se for the production of a pharmaceutical composition for the *in vivo* suppression of transplant rejection.

Such pharmaceutical preparation may contain vital TAIC according to the invention, which are obtained from step d) of the process of the invention, suspended in a pharmaceutically acceptable carrier preferably in a quantity of about 1 × 10⁵ to 1 × 10⁷ cells/ml and more preferably of about 1 × 10⁶ cells/ml of the preparation.

The cells of the TAIC_{GM7} sub-population are used per se for the production of a pharmaceutical composition for the *in vivo* suppression of transplant rejection.

Such pharmaceutical preparation may contain vital TAIC_{GM7} cells, which bind to the antibody GM-7, suspended in a pharmacologically acceptable liquid carrier, preferably in a quantity of about 1 × 10⁶ to 1 × 10⁸ cells/ml and more preferably of about 1 × 10⁶ cells/ml of the preparation.

The cells of the TAIC_{CD3+/CD14+} sub-population are used per se for the production of a pharmaceutical composition for the *in vivo* suppression of transplant rejection.

Such pharmaceutical preparation may contain vital TAIC_{CD3+/CD14+} cells which co-express the antigens CD3 and CD14, in a quantity of preferably about 5 × 10⁵ to 5 × 10⁷ cells/ml and more preferably of about 5 × 10⁶ cells/ml of the preparation.

The above described pharmaceutical preparations may contain the cells according to the invention suspended in a physiologically well-tolerated medium. Suitable media are for example Ringer solution, physiological saline or 5 to 20 % human albumin solution and the like.

Cell preparations according to the invention may contain vital TAIC, which are obtained from step d) of the process of the invention. Alternatively, the preparations may contain cells belonging to the sub-populations of TAIC_{GM7} cells, which bind to the antibody GM-7, or of TAIC_{CD3+/CD14+} cells, which co-express the antigens CD3 and CD14 on their cell surface. The preparations may contain the respective cells in a quantity of preferably at least 1 × 10⁵, more preferably at least 5 × 10⁵ and most preferably at least 1 × 10⁶ cells per ml suspended in a liquid carrier medium. The medium may be a cell culture or transport medium well-tolerated by cells, such as 5 to 20% human albumin solution. Alternatively, the cells within the preparation may be deep-frozen and contained in a suitable storage medium, such as e.g. RPMI with 50% human albumin solution and 10% DMSO.

Finally, the invention also relates to a method wherein the transplant-acceptance inducing cells of the invention (TAIC, TAIC_{GM7} or TAIC_{CD3+/CD14+}) are used for generating or expanding regulatory T-lymphocytes *in vitro*. As shown in Example 12, the direct *in vitro* cocultivation of TAIC with lymphocytes leads to a significant proliferation of regulatory T-lymphocytes, in particular of CD4⁺/CD25⁺ lymphocytes, see Wood and Sakaguchi: "Regulatory Cells in Transplantation Tolerance", Nature Review Immunology 3, 199-210 (2003). It is therefore possible to generate and/or expand regulatory T-lymphocytes in particular CD4⁺/CD25⁺ lymphocytes by directly coculturing TAIC with lymphocytes as described in Example 12.

Direct *in vitro* culturing means according to the invention that TAIC and lymphocytes are cocultured in direct physical contact within the same medium, as a.o. exemplified in Example 12.

In this method the medium preferably contains the respective cells i.e. TAIC and lymphocytes in about equal cell numbers and each in a quantity of preferably at least 1 × 10⁵, more preferably at least 5 × 10⁵ and most preferably at least 1 × 10⁶ cells per ml suspended in a liquid carrier medium; the medium may be a cell culture or transport medium well-tolerated by cells, such as 5-20% human albumin solution. The co-cultivation preferably should take place under physiological conditions at about 37°C, e.g. in an incubator, for preferably about 3 to 5, more preferably 4 days.

As shown in Example 13, transplant acceptance can not only be induced by administering TAIC generated from donor monocytes to the recipient, but also by readministering recipient lymphocytes to the recipient, which have previously been directly cocultured *in vitro* with TAIC prepared from donor monocytes as described above.

According to a further embodiment of the invention regulatory T-lymphocytes can therefore be prepared *in vitro* from lymphocytes originating form the recipient of a transplant, by direct coculturing of lymphocytes from the recipient with TAIC originating from the donor of that transplant. Readministering the cocultivated lymphocytes to the recipient will lead to transplant acceptance by the recipient, as shown in Example 13.

The recipient derived regulatory T-lymphocytes so prepared may be isolated by FACS as described herein (see above) and may be used in a pharmaceutical preparation for preventing transplant rejection in the recipient, wherein the cells are suspended in a pharmacologically acceptable carrier as described above.

The invention is illustrated in further detail by way of examples.

If not defined within the examples, the composition of the media and substances used are as follows:
**1. Penicillin/streptomycin solution:**
10,000 units of penicillin as sodium salt of penicillin G and 1000 µg streptomycin as streptomycin sulphate per ml physiological sodium chloride solution (NaCl 0,85 %) (Gibco Catalogue No. 15140122).
**2. Trypsin-EDTA**
0.5 g trypsin and 0.2 g EDTA (4 Na)/l
**3. RPMI 1640 (1x, liquid (11875))**
**contains L-Glutamine**
RPMI (Roswell Park Memorial Institute) Media 1640 are enriched formulations, which can be used extensively for mammalian cells.

| **Components** | **Mol.-weight** | **Conc. (mg/l)** | **Molarity (nM)** |
|---|---|---|---|
| **Anorganic salts** | | | |
| Calcium nitrate (Ca(NO₃)₂ 4H₂O) | 236 | 100.00 | 0.424 |
| Potassium chloride (KCI) | 75 | 400.00 | 5.30 |
| Magnesium sulphate (MgSO₄) | 120 | 48.84 | 0.407 |
| Sodium chloride (NaCl) | 58 | 6000.00 | 103.44 |
| Sodium bicarbonate (NaHCO₃) | 84 | 2000.00 | 23.800 |
| Sodium phosphate (Na₂HPO₄) | 142 | 800.00 | 5.63 |

| **Further components** | | | |
|---|---|---|---|
| Glucose | 180 | 2000.00 | 11.10 |
| Glutathione, reduced | 307 | 1.50 | 0.0032 |
| Phenol red | 398 | 5.00 | 0.0125 |

| **Amino acids** | | | |
|---|---|---|---|
| L-Arginine | 174 | 200.00 | 1.10 |
| L-Asparagine | 132 | 50.00 | 0.379 |
| L-Asparaginic acid | 133 | 20.00 | 0.150 |
| L-Cysteine dihydrochloride | 313 | 65.00 | 0.206 |
| L-Glutamininc acid | 147 | 20.00 | 0.136 |
| L-Glutamine | 146 | 300.00 | 2.05 |
| Glycine | 75 | 10.00 | 0.133 |
| L-Histidine | 155 | 15.00 | 0.0967 |
| L-Hydroxyproline | 131 | 20.00 | 0.153 |
| L-Isoleucine | 131 | 50.00 | 0.382 |
| L-Leucine | 131 | 50.00 | 0.382 |
| L-Lysine hydrochloride | 146 | 40.00 | 0.219 |
| L-Methionine | 149 | 15.00 | 0.101 |
| L-Phenylalanine | 165 | 15.00 | 0.0909 |
| L-Proline | 115 | 20.00 | 0.174 |
| L-Serine | 105 | 30.00 | 0.286 |
| L-Threonine | 119 | 20.00 | 0.168 |
| L-Tryptophan | 204 | 5.00 | 0.0245 |
| L-Tyrosine disodium, dihydrate | 261 | 29.00 | 0.110 |
| L-Valine | 117 | 20.00 | 0.171 |

| **Vitamins** | | | |
|---|---|---|---|
| Biotin | 244 | 0.20 | 0.008 |
| D-calcium pantothenate | 477 | 0.25 | 0.0005 |
| Choline chloride | 140 | 3.00 | 0.0214 |
| Folic acid | 441 | 1.00 | 0.0022 |
| i-Inositol | 180 | 35.00 | 0.194 |
| Niacinamide | 122 | 1.00 | 0.0081 |
| p-aminobenzoic acid (PABA) | 137 | 1.00 | 0.0072 |
| Pyridoxine HCl | 206 | 1.00 | 0.0048 |
| Riboflavin | .376 | 0.20 | 0.0005 |
| Thiamin HCl | 337 | 1.00 | 0.0029 |
| Vitamin B12 | 1355 | 0.005 | 0.00000369 |

| | | | |
|---|---|---|---|
| Reference: Moore G.E., et al., J.A.M.A. **199**: 519 (1967) | | | |

**4. PBS (Dulbecco's phosphate buffered saline)** cf. J. Exp. Med. 98:167 (1954):

| Components | g/l |
|---|---|
| KCl | 0.2 |
| KH₂PO₄ | 0.2 |
| NaCl | 8.00 |
| Na₂PHO₄ | 1.15 |

**5. Ficoll-Hypaque:**
Lymphocyte separation medium (saccharose/epichlorohydrin-copolymerisate Mg 400,000; Density 1.077, adjusted with Sodium diatrizoate).
**6. L-Glutamine**
Liquid: 29.2 mg/ml
**7. Macrophage-colony stimulating factor (M-CSF)**
Recombinant human M-CSF from E. coli; contains as monomer (18.5 kD) 135 amino acid residues including the N-terminal methionine; is present as a homodimer with a molar mass of 37 kD; (SIGMA Catalogue No. M 6518)
**8. γ-Interferon (γ-IFN)**
Recombinant human γ-IFN from E. coli; 16.7 kD protein containing 143 amino acid residues (CHEMICON Catalogue No. IF002)

### Example 1

### Separation of Monocytes from whole Blood

To avoid the coagulation of the blood and to feed the cells, 450 ml human whole blood was mixed in a triple chamber bag set with 63 ml of a stabiliser solution which contained, per litre H₂O, 3.27 g of citric acid, 26.3 of g trisodium citrate, 25.5 g of dextrose and 22.22 g of sodium dihydroxy phosphate. The pH of the solution was 5.6-5.8.

To separate the components of the blood, "sharp centrifuging" of this mixture was subsequently carried out at 4000 rpm for 7 minutes at 20°C. This resulted in the layering of the corpuscular and non-corpuscular components within three layers. By using the bag set in a compression machine provided for this purpose, the erythrocytes were then pressed into the lower bag, the plasma was pressed into the upper bag and the so-called buffy-coat remained in the middle bag and contained a volume of approximately 50 ml.

The quantity of 50 ml of freshly obtained buffy-coat was then divided into 2 portions of 25 ml each and layered onto 25 ml of Ficoll-Hypaque separation medium respectively which had been introduced previously into two 50 ml Falcon tubes.

This preparation was centrifuged for 30 minutes at 2500 rpm without braking. Thereafter, any erythrocytes and dead cells still present in the buffy-coat were below the Ficoll phase, whereas the white blood cells, including the monocytes, were separated off on the Ficoll as white interphase.

Subsequently, the white interphase including the monocytes was carefully removed by pipetting and mixed with 10 ml of phosphate-buffered saline (PBS).

This preparation was then centrifuged three times for 10 minutes at 1800 rpm with braking, the supernatant being removed by pipetting after each centrifuging process and fresh PBS being introduced.

The cell pellets assembled at the bottom of the centrifuging vessel (Falcon tubes) contained the mononuclear cell fraction, i.e. the monocytes.

To prepare the monocytes required for the rat experiments, 25 ml of whole blood were isolated from four genetically identical donor rats respectively and each was layered onto a 25 ml Ficoll-Hypaque separation medium. The procedure was then continued in the same way as described above. Alternatively, analogous separation steps on Ficoll may also be applied to spleen cell suspensions.

### Example 2

### Propagation and Modification of the Monocytes

The cultivation and propagation of the monocytes was carried out in nutrient medium with the following composition:

| | |
|---|---|
| RPMI 1640 medium | 440 ml |
| Fetal calf serum (FCS), alternatively, AB serum | 50 ml |
| Penicillin / Streptomycin Solution | 5 ml |
| Total volume | 500 ml |

The nutrient medium contained 2,5 µg / 500 ml M-CSF.

The monocytes isolated in Example 1 were suspended in a total quantity of 10⁶ cells in 10 ml of the nutrient medium and transferred onto a Petri dish (100 mm in diameter). The Petri dish had previously been filled with pure inactivated FCS and the FCS had been poured off after 24 hours in order to obtain, in this way, a dish coated with FCS.

The Petri dish was covered with the appropriate cover and kept for 3 days in an incubator at 37°C. The cells settled at the bottom of the Petri dish after 24 hours. On day two, the supernatant was pipetted off and the Petri dish again filled with 10 ml of fresh nutrient medium.

On day 4, 50 ng γ-interferon in 10 ml nutrient medium were added and the dish was again closed and kept for a further 48 hours in the incubator at 37°C.

Subsequently, 10 ml of trypsin solution each diluted 1:10 with PBS were pipetted into the Petri dish. The closed Petri dish was kept for 10 minutes in the incubator at 37°C.

Thereafter, the separation of the cells adhering to the bottom of the Petri dish was carried out with a cell scraper in such a way that the major part (>90%) of the cells floated in the supernatant.

The total supernatant (10 ml of trypsin solution + 10 ml of medium) was pipetted off, combined in a 50 ml Falcon tube and centrifuged for 10 minutes at 1800 rpm. The supernatant was then discarded and fresh nutrient medium (see above) was added to the precipitate (the remaining cell pellet), 1 ml of nutrient being added per 10⁶ cells. The determination of the cell count for the determination of the exact dosage took place according to known methods, compare Hay R.J., "Cell Quantification and Characterisation", in Methods of Tissue Engineering, Academic Press 2002, Chapter 4, S. 55-84.

This cell suspension was centrifuged (1800 rpm, 10 minutes, see above) and the cell pellet were incorporated either into PBS or for application in man into NaCl (physiol.). Subsequently, the intravenous administration can take place directly or within 48 hours.

Alternatively, after centrifuging and discarding the supernatant containing trypsin, FCS/DMSO was added as freezing medium to the cells and these were deep frozen in a volume of 10 ml.

The freezing medium contained 95% FCS and 5% DMSO. In each case, approximately 10⁶ cells were incorporated into 1 ml of the medium and cooled in the following steps:
30 minutes on ice;
2 hours at -20°C in the pre-cooled Styropor box;
24 hours at -80°C in Styropor;
Storage in small tubes in liquid nitrogen (N₂) at -180°C.

Figure 1 shows the phenotypic changes in the antigen expression, determined by flow cytometry, on the monocytes used after cultivation and γ-IFN stimulation of the original monocytic cells.

### Example 3

### Use of the Transplant Acceptance Inducing Cells (TAIC) for Preconditioning of the Immune System of the Transplant Recipient

The use of TAIC for treating a potential recipient before transplantation offers itself, for example, in the clinical case of a live donation, where it has been clarified in advance of the organ transplantation who the donor will be and who will receive the donor organ.

For this case, the heterotopic heart transplantation was carried out in the rat model on male inbred rats of the strain combination LEW[RT1.¹] (in connection with the present invention referred to as "LEW") -> DA[RT1.^{av1}] (in connection with the present invention referred to as "DA") using the technique described by Ono and Lindsey [Ref. Ono, K. and Lindsey, E.S. "Improved technique of heart transplantation in rats". J. Thorac. Cardiovasc. Surg. 57, 225-229 (1969)]. On day -7 before the transplantation, the DA recipient rats received 10⁶ TAIC derived from LEW monocytes intravenously in 1 ml of PBS. Subsequently, the transplantation took place seven days later of a heart taken from the LEW rat strain, which was heterotopically implanted into the abdomen (abdominal cavity) of the DA recipient animal. Since the rat strains used were inbred strains, the TAIC according to the invention expressed identical tissue antigens as the transplanted LEW heart.

For control purposes, a so-called third strain transplantation was carried out. For this purpose, TAIC (10⁶) originating from the same LEW donor animal were injected intravenously into DA animals again on day -7 (i.e. 7 days before the surgical intervention). 7 days afterwards, the transplantation of a heart removed from a CAP[RT1.^{c}] (in connection with the present invention referred to as "CAP") rat inbred strain was carried out. CAP rats express the haplotype RT1.^{c}, they are therefore fully MHC-discordant (MHC-different) to the LEW donor animals. Below, the individual test groups in which a heterotopic heart transplant took place (n=10) are detailed:

| | | |
|---|---|---|
| 1. | LEW -> | DA; untreated; |
| 2. | LEW -> | DA; 10⁶ LEW derived TAIC i.v., day -7 before LEW heart transplantation; |
| 3. | CAP -> | DA; 10⁶ LEW derived TAIC i.v., day -7 before LEW heart transplantation; |
| 4. | CAP -> | DA; untreated; |
| 5. | LEW -> | LEW, untreated; |
| 6. | LEW -> | LEW, 10⁶ LEW derived TAIC i.v., day -7 before LEW heart transplantation. |

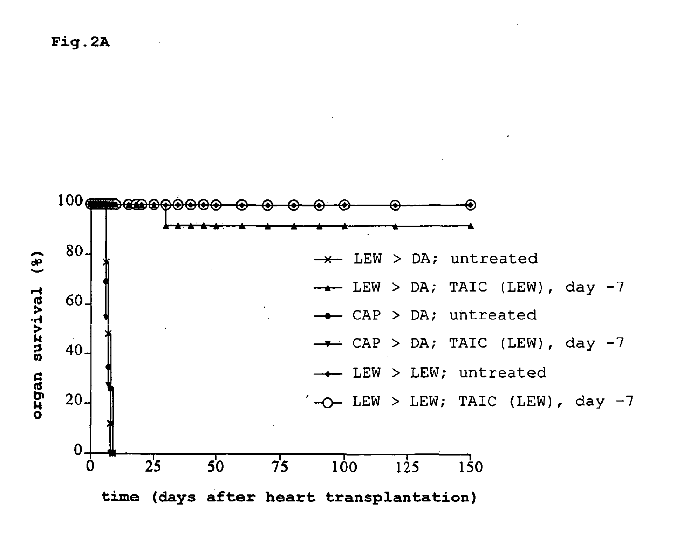

As can be seen from the Kaplan-Meier survival curves of the heart organs (Figure 2A), 9 of 10 LEW hearts were accepted long-term (>150 days) by DA recipient animals whereas CAP third strain hearts were rejected acutely after 6.7 ± 0.8 days. Where no prior administration of TAIC took place, the LEW hearts were also rejected acutely after 7.2 ± 1.0 days. The rejection of CAP hearts by the immune response stimulated in the DA animal after 6.7 ± 0.8 days took place within the same time span as in the untreated control-group in which the CAP hearts of DA rats were rejected after 6.9 ± 1.0 days, compare group 3 and group 4, respectively. All hearts transplanted syngeneically in the LEW -> LEW scheme survived long-term (>150 days) irrespective of whether or not the TAIC pretreatments had taken place. From this, the conclusion can be drawn that TAIC do not put the recipient organism at risk in the sense of a graft versus host disease. Moreover, it is possible to draw the conclusion from this result that the i.v. administration of 10⁶ TAIC 1 week before transplantation opens up the possibility of inducing long term organ acceptance. The induced tolerance is donor-specific since a corresponding pre-treatment of DA rats with LEW-derived TAIC does not induce tolerance for CAP third strain heart transplants. In the syngeneic LEW > LEW scheme, the additional administration of TAIC influences neither the survival of the recipient animals nor that of the transplants.

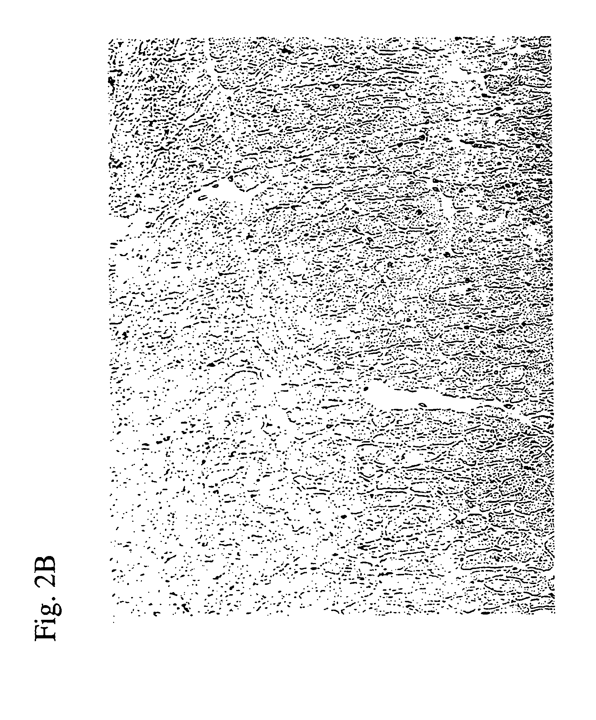

Figure 2B shows the histological evaluation of a LEW allogeneic transplant on postoperative day (POD) 150, following heterotopic transplantation into the abdomen of a DA recipient rat. The recipient rat had been pre-treated on day -7 before transplantation with 10⁶ LEW-derived TAIC. The transplant was functioning satisfactorily (strong heart beat) up to the time of its removal from the recipient rat (POD) 150. The histological preparation (x40) showed a healthy heart muscle morphology, normal vascular endothelial tissue and only minimal infiltration with mononuclear cells without any indication of acute and chronic rejection processes.

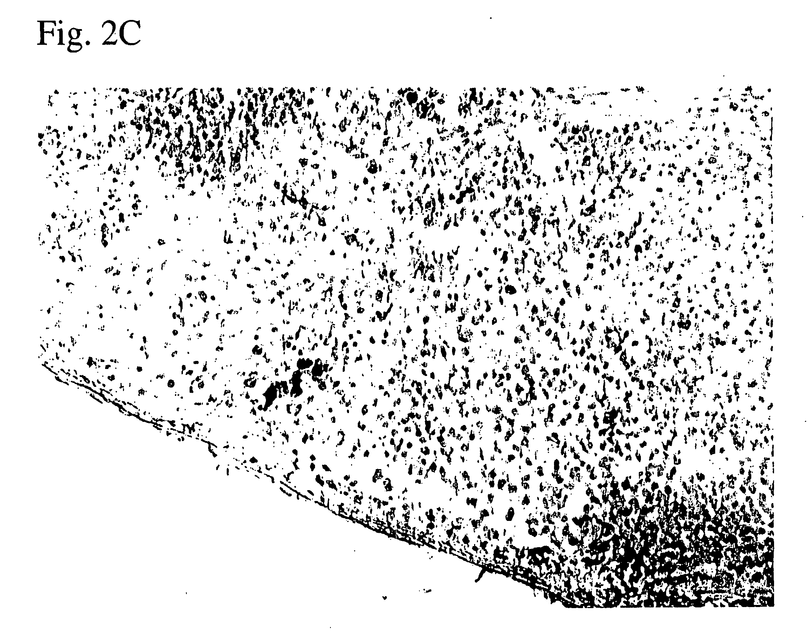

Remarkable in this connection is also the fact that donor-derived cells (detected by immunohistochemical detection using LEW-MHC class I-specific antibodies I1.69) could be detected long-term in the course of a year in the thymus of the DA recipient rats. Figure 2C shows a histological preparation of a thymus, removed on POD 150, of a DA rat pre-treated with 10⁶ LEW-derived TAIC. The preparation was labelled with the monoclonal antibody I1.69 specific for LEW-MHC class I. As shown in the Figure, administering TAIC leads to the colonisation of the thymus gland where the presentation of donor-derived MHC antigens to the maturing T-cells takes place; small nests of cells can be clearly recognised which can be labelled with the antibody.

### Example 4

### Detection of Donor-Derived Modified Monocytes in the Recipient

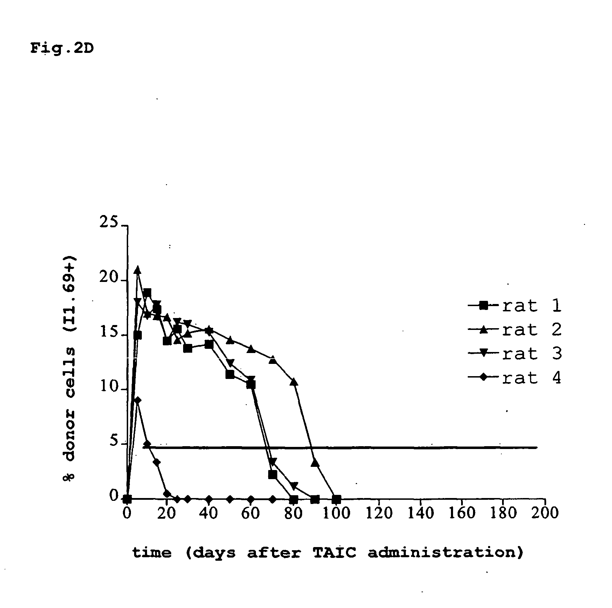

The induction of a mixed chimerism by the intravenous injection of the LEW-derived TAIC in the DA animal concerned can be considered as a possible cause of the tolerance induced according to the invention. A test was therefore carried out to check whether, following the injection of LEW-derived TAIC, more than 5% of cells can be detected in the blood of the recipient animal by flow cytometry in the long term (>45 days). The flow cytometry was carried out as described in the state of the art [Ref. Preffer F.I., "Flow cytometry" In: Diagnostic Immunopathology, Colvin RB, Bhan AK, McCluskey, RT (eds.), Raven Press New York, pp. 725-449 (1994)]. The detection of the LEW donor cells in the DA recipient blood took place using the monoclonal antibody I1.69 which specifically detects the LEW-MHC class I antigens [see Fändrich F., et al. Nature Med. 8, 171-178, (2002)]. The chimerism data which were determined for 4 of the LEW -> DA transplantations described in Example 3, following the administration of 10⁶ or 10⁴ TAIC / kg × BW respectively, in the peripheral blood of the recipient animals are shown in Figure 2D.

Following the injection of LEW-derived TAIC into four DA recipient rats not subjected to immunosuppression (10⁶ cells / kg × BW into rats 1-3 and 10⁴ cells / kg × BW into rat 4), rats 1-3 showed a longer term chimerism (60-80 days) in the DA recipient blood, which could be attributed to donor-derived cells. The detection of donor-derived cells took place by flow cytometric determination using the LEW-MHC class I- specific antibody I1.69 (a monoclonal antibody which binds only to MHC class I positive cells of the LEW strain of rat and does not cross-react with cells of DA strains of rat). Since quasi all cells of the peripheral blood express MHC class I antigen on the cell surface, the proportion of donor cells in the peripheral blood of the DA rat can be highly accurately determined with I1.69. As shown in the graphs in Figure 20, in 3 of 4 animals, more than 10% of the cells express the donor antigen labelled by I1.69 within the first 6 weeks following the intravenous injection of the TAIC. After 60 days following injection, this proportion of the chimerism in the peripheral blood of these DA rats decreases substantially and disappears completely on day 100. However, this transient chimerism correlates closely to the transplant survival of LEW hearts transplanted after double time (i.e. 7 days after TAIC injection) which are no longer rejected in spite of a lack of donor chimerism (after day 100) (rats 1-3 in the example). On the other hand, the induction of short-term chimerism following the administration of 10⁴ cells / kg × BW (Rat 4; <20 days) does not guarantee the long-term transplant acceptance of the transferred heart.

### Example 5

### Administration of Donor-Derived Cells post transplantationem

An evaluation was then to be carried out for the clinical case of corpse (deadbody) donation to determine to what extent the postoperative administration of TAIC was capable of inducing the corresponding long-term acceptance of the transplanted organ in the recipient. For this purpose, heterotopic heart transplantations were carried out in the LEW -> DA rat inbred strain model. The DA recipient rats received 4 cycles of cyclosporin A (CSA) intravenously in the dosages of 5 mg CSA / kg × BW on days 0, 1, 2 and 3 following transplantation. On day 7, the intravenous administration of 10⁶ LEW-derived TAIC intravenously into the tail vein of the DA recipient rats took place. The following experimental groups served as controls (n=6-10).

| | | |
|---|---|---|
| 1. | LEW -> | DA; untreated |
| 2. | LEW -> | DA; CSA, 5 mg / kg × BW, i.v., day 0, 1, 2 and 3 |
| 3. | LEW -> | DA; CSA, 5 mg / kg × BW, i.v., day 0, 1, 2 and 3 plus 10⁶ LEW-derived TAIC, i.v., day 7 |
| 4. | LEW -> | DA; 10⁶ LEW-derived TAIC, i.v., day 7 |
| 5. | CAP -> | DA; CSA, 5 mg / kg × BW, i.v., day 0, 1, 2 and 3 plus 10⁶ LEW-derived TAIC, i.v., day 7 |

The postoperative intravenous treatment of the DA recipient rats with 10⁶ LEW-derived TAIC on POD (postoperative day) 7 leads to long-term tolerance if an initial administration of 4 cycles of CSA (5mg/kg × BW, i.v.) has been effected from day 0-3. The initial administration of CSA is necessary since the sole treatment with a single administration of 10⁶ TAIC on POD 7 (postoperative day) prevents acute rejection only in 1 out of 6 cases. The CSA dose chosen in this case has only a sub-therapeutic effect since no long-term acceptance can be induced by the exclusive CSA administration even though the survival of the transplant is prolonged. The induced transplant acceptance is donor-specific since a corresponding treatment of DA rats with LEW-derived TAIC does not induce any tolerance for CAP-third strain heart transplants.

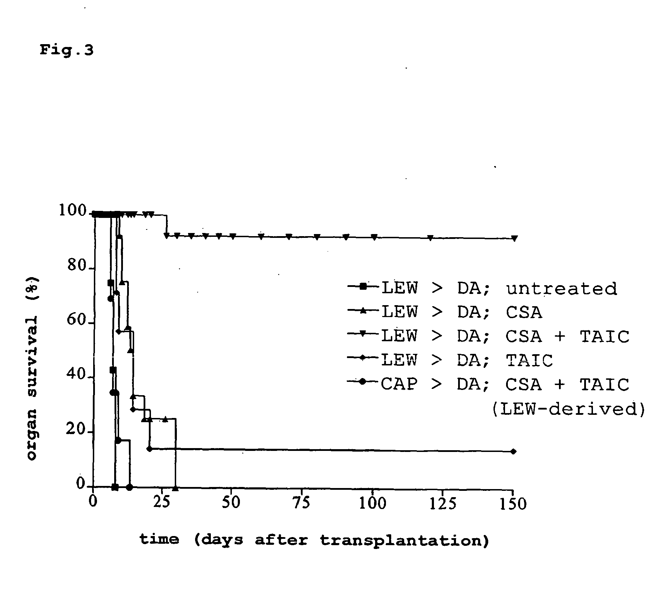

As shown in Figure 3, the hearts of experimental group 1 were rejected acutely after 7.0 ± 0.8 days. The hearts of group 2 were rejected with a delay after 14.4 ± 7.0 days. The hearts of group 3 were accepted for longer than 150 days in 5 out of 6 cases. In the rat system, the acceptance of organs for more than 100 days is considered to be long-term acceptance since the average life expectancy of an inbred rat is only approximately 2 years. The sole postoperative administration of TAIC (10⁶ cells) was able to prevent acute rejection only in 1 out of 6 cases since the heart organs ceased to beat after 22.6 ± 31.6 days (group 4). The third strain control with CAP hearts showed the specificity of induced transplant acceptance since third strain hearts were again rejected acutely after 7.4 ± 2.2 days. These results also prove that the additional application of TAIC cannot generate any general immunosuppression which did not prevent the acute rejection to the CAP hearts.

### Example 6

### Investigation regarding the Organ Specificity of the Long-Term Acceptance induced by TAIC

To exclude the possibility of TAIC being able to provide a protective effect against the allogeneically specific immune response directed by the recipient against the donor antigen only for heart transplants, the following organs were transplanted in the following experimental groups (n=6) in the strongly rejecting DA -> LEW rat inbred system:

| | | |
|---|---|---|
| 1. | DA (kidney) -> | LEW; untreated |
| 2. | DA (kidney) -> | LEW; 10⁶ DA-derived TAIC, i.v., day -7 and -1 before transplantation |
| 3. | LEW (kidney) -> | LEW; untreated |
| 4. | DA (liver) -> | LEW; untreated |
| 5. | DA (liver) -> | LEW; 10⁶ DA- derived TAIC, i.v., day -7 and -1 before transplantation |
| 6. | LEW (liver) -> | LEW; untreated |
| 7. | DA (skin) -> | LEW; untreated |
| 8. | DA (skin) -> | LEW; 10⁶ DA- derived TAIC, i.v., day -7 and -1 transplantation |
| 9. | LEW (skin) -> | LEW; untreated |

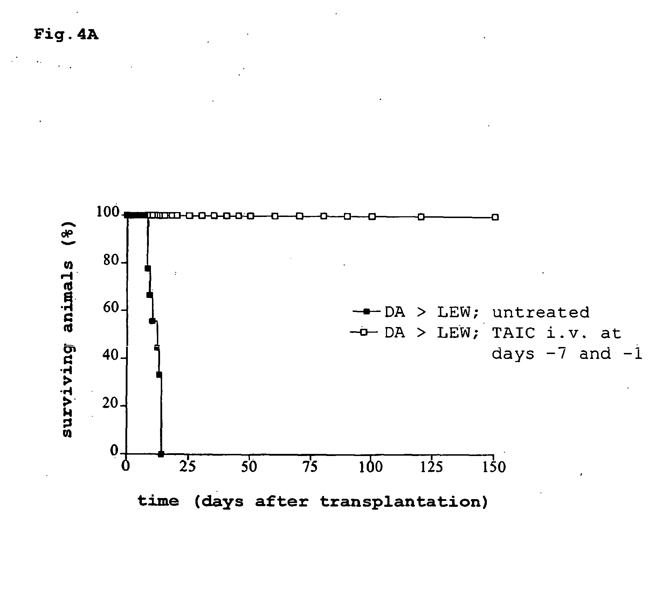

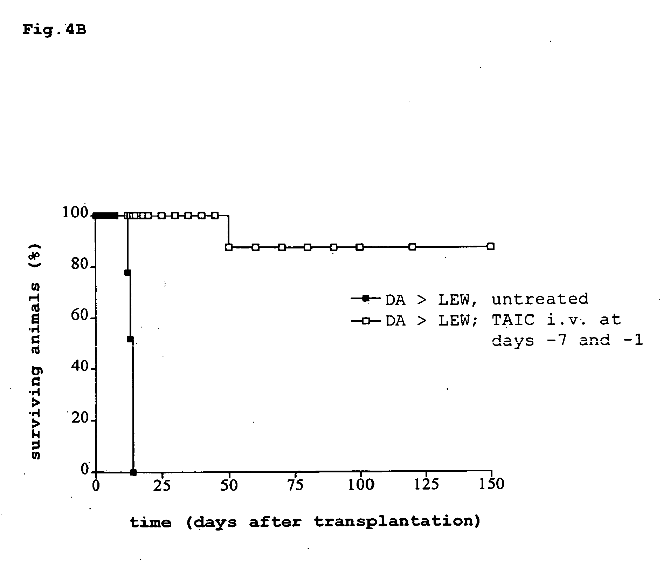

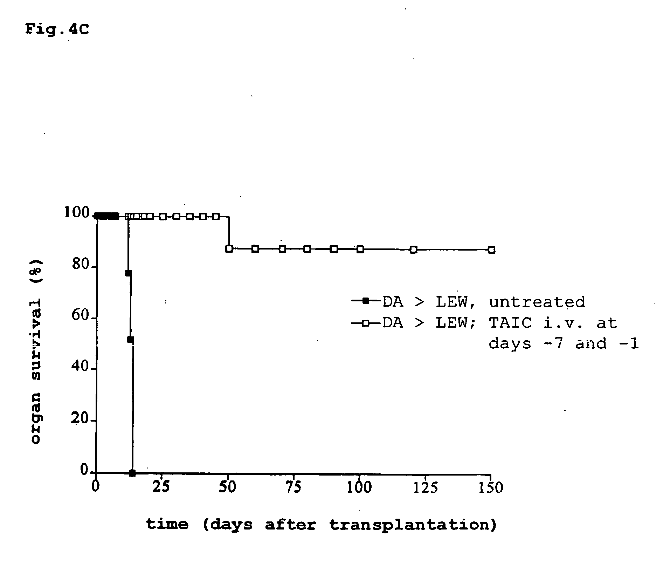

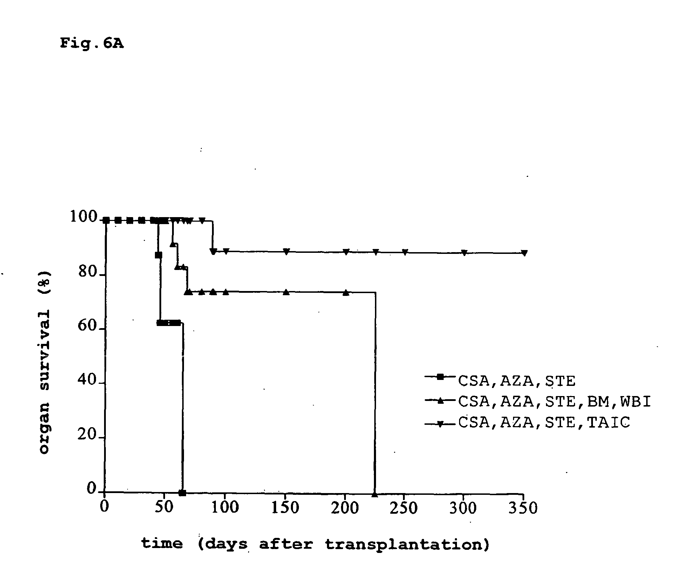

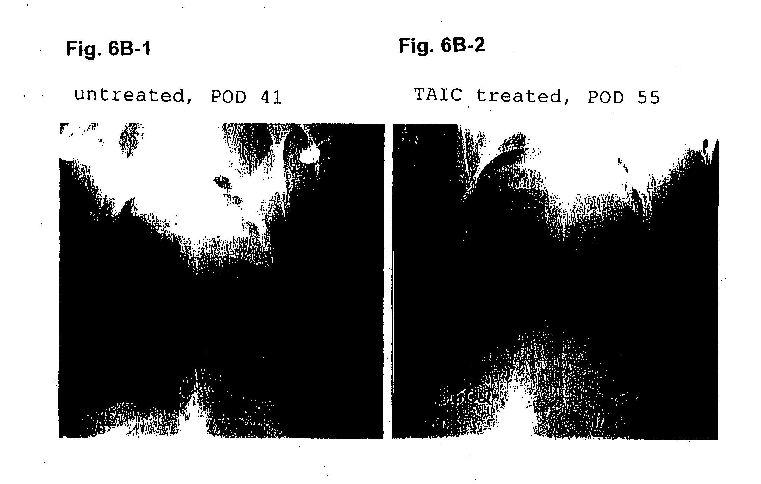

As shown by the Kaplan-Meier plots illustrated in Figure 4A-C, 6 out of 6 kidney organs, 5 out of 6 liver organs and 5 out of 6 skin grafts were accepted long term (>150 days) by LEW recipient rats. All syngeneic organ transplantations served as controls of the technical execution without complications and were accepted by the donor animal without restriction (not shown in the survival curves). Consequently, these test schemes also show that the intravenous prior injection of 2 × 10⁶ TAIC induces long-term organ acceptance for more than 80% of the transplanted organs.

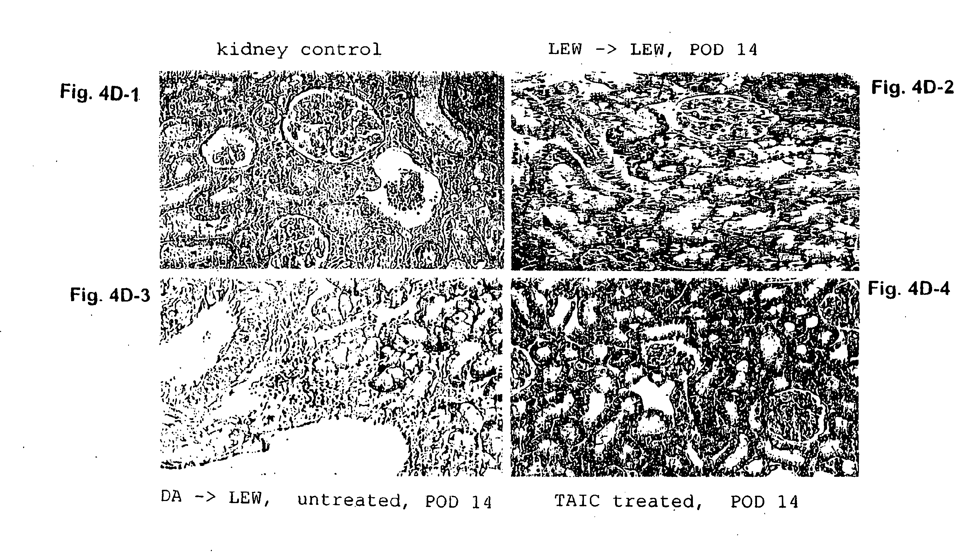

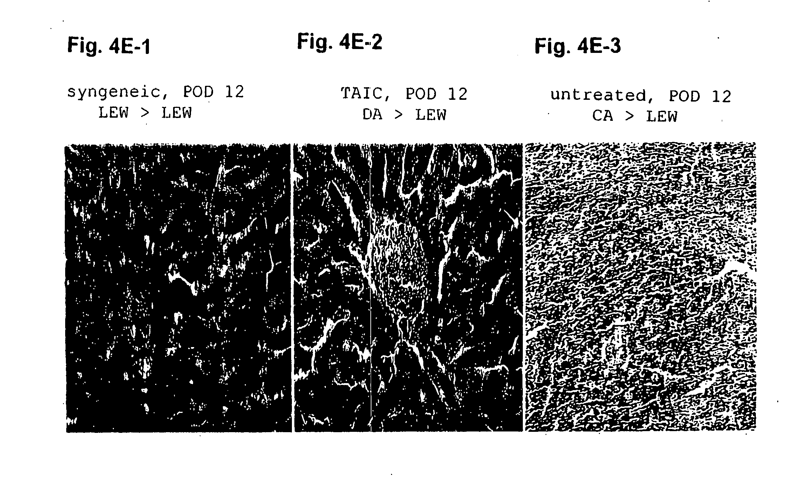

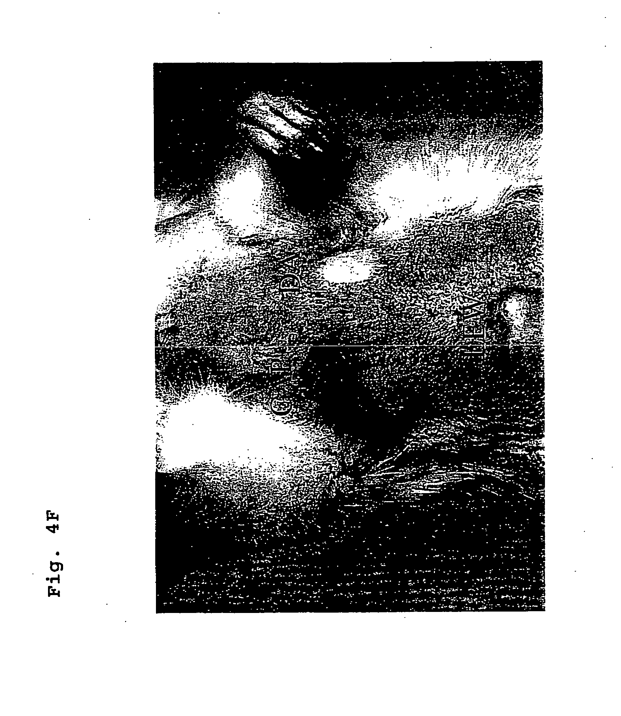

The kidney-, liver- and skin transplants listed above which were transplanted with and without the prior administration of TAIC were then examined histologically. Figure 4D shows that as a result of the intravenous injection, given twice, of 10⁶ TAIC per kg body weight on days -7 and -1 before transplantation, the morphology of the transplanted kidney can be shown to be intact (compare control kidney and syngeneic transplant LEW -> LEW), whereas, in the untreated allogeneic control group DA -> LEW, a clear infiltration of the transplant in the interstice can be recognised (on postoperative day 14). The allogeneic liver transplant (Figure 4E), which had been fully rejected on POD 12 without TAIC pre-treatment (DA -> LEW without TAIC), behaves in an analogous manner whereas the prior administration of TAIC allows the parenchyma of liver transplants to appear as being completely homogeneous and intact in comparison with the syngeneic transplant. The allogeneic skin transplant (DA) also heals without complication (compare comparison with the syngeneic LEW transplant) whereas the CAP third strain graft transplanted simultaneously was rejected acutely as early as on postoperative day 12. This example proves the specificity of the tolerance induced by TAIC since the pre-treatment of the LEW recipient animal with DA-derived TAIC on days-7 and -1 leads to the acceptance of the DA transplant whereas the CAP skin graft transplanted simultaneously on day 0 is acutely rejected (Figure 4F).

### Example 7A

### Investigations regarding the Haplotype Specificity of the Long-Term Organ Acceptance induced by TAIC

In order to exclude the possibility of TAIC inducing long-term organ acceptance only in the selected rat inbred strain combination LEW -> DA, the following further inbred strain combinations were examined in the heterotopic heart transplant model (n=6). For this purpose, the inbred strain BN[RT1.ⁿ] (referred to as "BN" in connection with the present invention) was also used, among others.

| | | |
|---|---|---|
| 1. | DA -> LEW; | untreated |
| 2. | DA -> LEW; | CSA, 5 mg / kg × BW, i.v., day 0, 1, 2, 3 plus 10⁶ DA-derived TAIC / kg × BW, i.v., day 7 and 10 postoperative |
| 3. | CAP -> LEW; | untreated |
| 4. | CAP -> LEW; | CSA, 5 mg / kg × BW, i.v., day 0, 1, 2, 3 plus 10⁶ CAP-derived TAIC / kg × BW, i.v., day 7 and 10 |
| 5. | BN -> LEW; | untreated |
| 6. | BN -> LEW; | CSA, 5 mg / kg × BW, i.v., day 0, 1, 2, 3 plus 10⁶ BN-derived TAIC / kg × BW, i.v., day 7 and 10 |

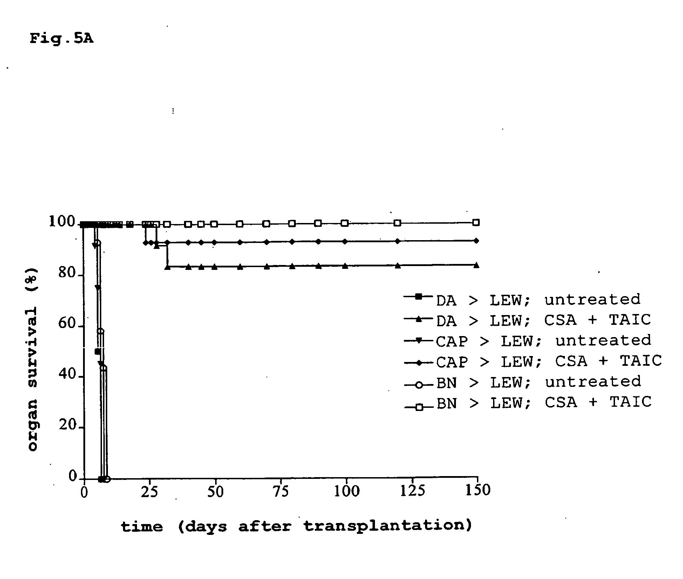

It had already been known from preliminary experiments that in the so-called "high-responder" combination (DA -> LEW), which is associated with the strongest rejection response known in the rat inbred model, no long-term acceptance can be achieved with the single postoperative i.v. administration of 10⁶ TAIC. An attempt was therefore made to increase the presence of TAIC in the recipient animal by administering it twice. As shown by the corresponding survival curves illustrated in Figure 5A, the administration of TAIC given twice on postoperative days 7 and 10 provided a long-term organ acceptance in 4 out of 6 cases in group 2 and in 5 out of 6 cases in experimental groups 4. In the BN -> LEW strain combination, all transplanted organs survived long-term without any indication of an acute rejection. In all cases in which the transplanted hearts had been accepted long term (>150 days), a mixed chimerism could be detected in the recipient animals during the first 20-45 days following the TAIC administration. An early organ failure, however, was always accompanied by a lack of donor cells in the peripheral blood of the recipient. From these data, the conclusion can be drawn that the i.v. administration of TAIC leads to long-term organ acceptance only if the cells induce a mixed donor chimerism which can be detected for at least 21 days. This also confirms the experimental results shown in Figure 2D.

By means of the postoperative administration of 10⁶ TAIC per kg body weight given to the recipient animals intravenously on days 7 and 10, it was consequently possible to induce long-term tolerance in all 3 inbred strain combinations in more than 80% of cases, if, additionally, 4 cycles of CSA were administered initially.

### Example 7B

### Investigations regarding the Significance of the γ-IFN Stimulation of M-CSF-treated Monocytes for in vivo Tolerance Induction

In order to be able to characterise the efficiency of the additional stimulation of M-CSF-treated blood monocytes with γ-interferon on the tolerance development *in vivo* in further detail, the following test groups were examined in the LEW -> DA rat inbred model for heterotopic heart transplantation (n = 6 animals per group):

| | | |
|---|---|---|
| 1. | LEW -> DA; | 10⁶ / kg × BW LEW-derived blood monocytes (uncultivated monocytes), i.v., day -7 before heart transplantation |
| 2. | LEW -> DA; | 10⁶ / kg × BW LEW-derived blood monocytes (treated with M-LSF), i.v., day -7 before heart transplantation |
| 3. | LEW -> DA; | 10⁶ / kg × BW LEW-derived blood monocytes (stimulated with γ-IFN), i.v., day -7 before heart transplantation |
| 4. | LEW -> DA; | 10⁶ / kg × BW LEW=derived blood monocytes (treated with M-CSF plus stimulated with γ-IFN), i.v., day -7 before heart transplantation |

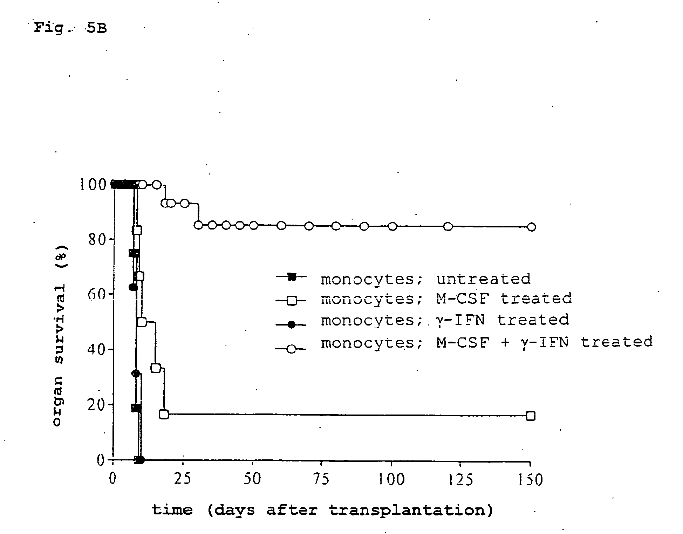

As shown by the Kaplan-Meier survival curves of Figure 5B, the intravenous administration of fresh non-cultivated LEW-derived blood monocytes, 7 days before heart transplantation, could not prevent the acute allogeneic transplant rejection of the LEW hearts in the DA donor animal. The transplants of this experimental group were rejected on average (± SD) after 7.8 + 0.8 days. The prior administration of monocytes cultivated over 6 days with M-CSF, however, resulted in a significant extension of the organ survival time since, in this experimental group, a rejection was observed on average only after 35.0 ± 51.5 days. The sole stimulation of fresh blood monocytes with γ-IFN over 48 hours in RPMI medium containing no M-CSF could not prevent the acute rejection of the heart transplants which ceased to beat on average after 7.8 ± 1.2 days. Only the M-CSF treatment of fresh blood monocytes over 6 days and the additional stimulation of these cells with γ-IFN over 48 hours (on day 5 and 6) led to long-term organ tolerance (> 150 days) in 4 out of 6 transplants. The results show the decisive importance of the additional stimulation of the M-CSF treated monocytes with γ-IFN for the generation of *in vivo* tolerance.

### Example 8

### Investigations regarding the Species Specificity of long-term Organ Acceptance induced by TAIC

In order to exclude the possibility of TAIC effectively inducing a long-term organ acceptance only in the selected animal species of rat, cells of monocytic origin were isolated from the donor pig in a manner analogous to the method detailed in Example 2, whereby the stimulation with γ-IFN was effected by adding 1000 U/10 ml to the nutrient medium over 24 hours. Subsequently, the investigation for the effectiveness of TAIC to suppress the acute rejection was carried out in the model of the left side lung transplantation on "SLA-mismatched minipigs" which have a potency to reject their organs comparable to that of man. In this case, the left lung of a donor pig is removed and transplanted orthotopically into a genetically different recipient pig (namely into the left thoracic cavity following the removal of the left lung from the recipient pig). At the time of the lung removal, 500 ml of donor blood was removed simultaneously which served to prepare the TAIC from the monocytes of this pig.

The donor animals all had a weight of 30-35 kg.
The following experimental groups were established in the pig (n=4) :
1. CSA, 5 g / day, 50 mg azathioprin / day, 25 mg methyl prednisolone / day, for a total of 28 days
2. As in group 1, plus 10⁸ bone marrow cells (BM) of the donor pig / kg × BW recipient pig plus 4.5 Gy whole body irradiation (WBI) of the recipient pig
3. As in group 1, plus 10⁶ TAIC derived from the donor pig / kg × BW on postoperative days 7 and 10 following lung transplantation

As shown in Figure 6A, the rejection of the lung transplants in group 1 took place on average 50.3 ± 9.9 days following the termination of immunosuppression. The animals in group 2, on the other hand, rejected their organs after 102.3 ± 81.0 days. Triple immunosuppression in combination with TAIC treatment repeated twice prolongs the organ survival to 292 ± 135.5 days, 3 out of 4 animals exhibiting a long-term organ acceptance (>350 days) of their organs. In all cases of group 3, a mixed donor chimerism could be observed in the recipient blood for >21 days, whereas animals of group 1 and 2 exhibited only a short-term mixed chimerism for 6-8 days. Figure 6B shows the lung transplants of a pig of experimental group 1 (POD 41) and a pig treated with TAIC from experimental group 3 (55 days after transplantation; B). Whereas, in a projection with respect to the left thorax wall around the heart silhouette, a visible reduction in transparency (shading) can be seen as an indication of the rejected lung transplantation in the pig of experimental group 1, the thorax x-ray picture of the animal treated with TAIC shows an x-ray unobtrusive lung and transplant finding respectively, with a good delimitation with respect to the heart silhouette

### Example 9

### Binding of the Antibody GM-7 to TAIC

The monoclonal antibody GM-7 was generated by immunising mice with human TAIC which had been prepared as described in Example 2. The hybridoma cells producing the antibody were deposited at the "Deutsche Sammlung für Mikroorganismen" under the accession no. DSM ACC2542. The results reported below demonstrate that the antibody binds specifically to an antigen which is expressed only on CD14⁺ cells which had been subjected to the 6 day *ex situ* modification with M-CSF and 2 day γ-IFN stimulation according to the invention.

Figure 1 shows the binding capacity, determined by flow cytometry, of GM-7 to monocytic cells after *in vitro* modification, i.e. after transformation into TAIC. It can be seen that the CD14-positive monocytes obtained directly from buffy-coat do not bind the antibody GM-7 (the grey shaded cloud corresponds to the antibody control). In contrast, part of the monocytes express an antigen after cultivation in M-CSF and stimulation with γ-IFN, which antigen is recognised by the monoclonal antibody GM-7. After cultivation as described in Example 2, about 80% of the transformed monocytes are able to bind the monoclonal antibody GM-7.

In a further experiment, the suppressor activity of the CD14⁺/GM-7⁺ cells was compared with that of CD14⁺/GM-7⁻ cells in a mixed lymphocyte culture (MLC). The MLC was carried out as described by Kurnick, J.T. "Cellular Assays" in: Diagnostic Immunopathology, [Colvin R.B., Bhan A.K., McCluskey, R.U. (ed.), Raven Press, New York, pages 751-771 (1994)].

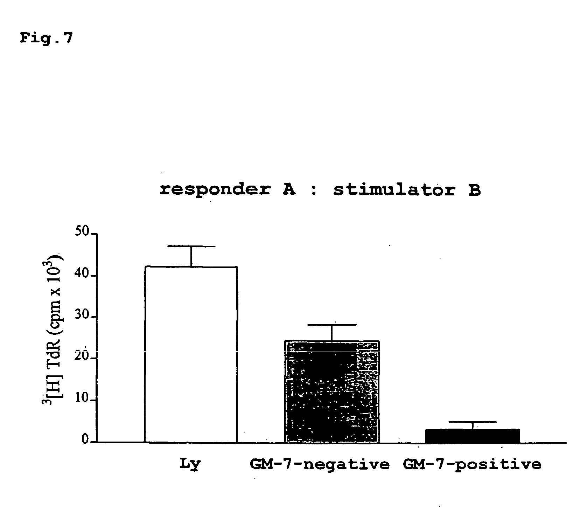

In this example, the TAIC stem from an individual B. As shown in Figure 7, a significant difference exist between the suppressor activity of the GM-7 positive and GM-7 negative TAIC. Only the GM-7 positive fraction the TAIC population obtained after 6 days treatment with M-CSF and 2 days stimulation with γ-IFN exhibits a significant inhibition effect on the T-cell proliferation activity of responder cells of the individual A after stimulation with cells of B.

These data indicate that, for clinical purposes, a cell population with optimised suppressor function can be isolated from the TAIC population obtained after cultivation of original monocytes with M-CSF and γ-IFN-stimulation through the availability of the monoclonal antibody GM-7.

### Example 10

### Determination of the Influence of the IDO Inhibitor 1-methyltryptophane on the Immunosuppressing Activity of the TAIC

In order to clarify, whether the inhibitor of the enzyme indolamine-2,3-dioxygenase (IDO) 1-methyltryptophane (1-MT) influences the suppressor function of the TAIC generated in the "Mo" and "Mo+Ly" set ups (see Example 11), a variety of mixed lymphocyte cultures (MLC) with PHA (phytohaemagglutinine) stimulated T-cells in the presence and in the absence of 1-MT were established.

In these mixed lymphocyte cultures 50.000 lymphocytes with 2 µg PHA were transferred into the wells of a 96-well-plate, followed by a proliferation period over 144 hours (indicated as "PhaLy"). Lymphocytes only cultivated in medium without addition of PHA were run as a further control (indicated as " "Ly").

In order to determine the proliferation of PHA-stimulated lymphocytes in the different co-cultures, the following 4 set ups were run and examined:

| | |
|---|---|
| PhaLy + "Mo+Ly" | PHA-stimulated lymphocytes and TAIC [10⁵ cells from the set up "Mo+Ly" according to Example 11]. |
| PhaLy+"Mo+Ly"+1-MT | PHA-stimulated lymphocytes and TAIC in the presence of 2 µmol 1-MT [10⁵ cells from the set up "Mo+Ly" according to Example 11]. |
| PhaLy + "Mo" | PHA-stimulated lymphocytes and TAIC [10⁵ cells from the set up "Mo" according to Example 11]. |
| PhaLy+"Mo"+1-MT | PHA-stimulated lymphocytes and TAIC in the presence of 2 µmol 1-MT [10⁵ cells from the set up "Mo" according to Example 11]. |

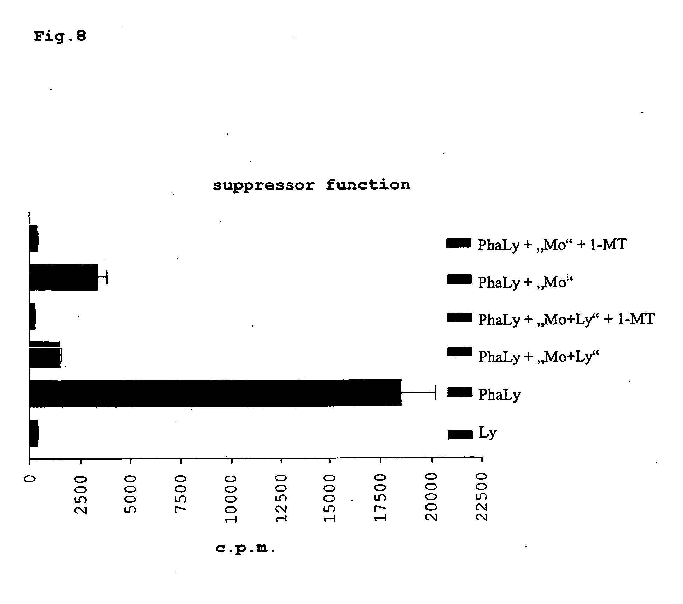

After 14.4 hours cultivation, all controls or co-cultures were further cultivated for 24 hours in the presence of ³[H]-thymidine ("pulsed") and thereafter the amount of radioactively labeled thymidine incorporated was determined as counts per minute (cpm), see Figure 8. In this arrangement, the amount of the radio activity determined is a measure for the amount of labeled thymidine incorporated into the DNA, and therefore a measure for the proliferation rate of the lymphocytes. The values reported in Figure 8 correspond to mean values from triple determinations of 3 experiments each with indication of the standard deviation.

The results demonstrated that lymphocytes not stimulated with PHA ("Ly") do not significantly proliferate, the mean radioactivity observed being 367 cpm (see Figure 8). In contrast, the stimulation with 2 µg PHA leads to a significant increase in the proliferation rate of the lymphocytes ("PhaLy"), the highest incorporation being measured at a mean value of 18459 cpm in these samples.

The addition of TAIC to the lymphocyte cultures clearly decreased the proliferation rate strongly if cells from the set up "Mo+Ly" from Example 11 were added (PhaLy + "Mo+Ly", determined value 1498 cpm), and less strongly when cells from the set up "Mo" from Example 11 were added (PhaLy + "Mo", measured value 3369 cpm).

The results obtained after addition of 2 µmol 1-methyltryptophane (1-MT) to the set ups containing "Mo+Ly" and "Mo" with stimulated lymphocytes demonstrated that 1-methyltryptophane (1-MT) increases the suppressor function of the TAIC synergistically, whereby the decrease was stronger with the TAIC from the set up "Mo+Ly" (measured value 267cpm) than with the TAIC from the set up "Mo" (measured value 390 cpm).

### Example 11

### Influence of Lymphocytes on the Formation of CD3⁺/CD14⁺-Cells during Monocyte Cultivation

The influence of lymphocytes present in the monocyte fraction on the generation of CD14⁺/CD3⁺-cells effective as TAIC was determined by comparison of two different set ups.

For the first set up (subsequently indicated as "Mo") the monocyte fraction was initially taken from the interphase of the buffy-coat as described in Example 1. As described in Example 2, the cells were subsequently transferred to the cultivation step with M-CSF. Within one hour after the starting point of the cultivation, the monocytes adhering to the bottom of the tissue culture flask were washed five times with 10 ml PBS each, and the amount of the lymphocytes present in the culture was thereby decreased to < 5 % (4,8 ± 2,4 %), while the amount of enriched monocytes (CD14⁺) so obtained was above 90 % (92 ± 5.6 %). Additional cell components within the set up were B-lymphocytes and granulocytes.

The cells in the second set up (subsequently indicated as "Mo+Ly") were also taken from the interphase of the "buffy-coat" as monocyte fraction as described in Example 1. However, different from set up "Mo" the cells adhering to the bottom of the tissue culture flask were only washed once after 24 hours starting from the beginning of the cultivation phase, as described in Example 2. As a result, a cell population was obtained, which was composed of 45 ± 5,3 % CD14⁺-monocytes and 23,5 ± 8,9 % CD2⁺-lymphocytes. As in set up "Mo" lymphocytes and granulocytes were also present in this set up.

The determination of the amounts of the respective cell types in the total cell population per set up was carried out through flow cytometry of three experimental set ups each (see Figure 9). The results are reported as mean values including the standard deviation.

CD14⁺/CD3⁺-cells can neither be determined in the set up "Mo" nor in the set up "Mo+Ly" at the beginning of the culture (d 0 = day 0). After a cultivation period of five days, the experiment was terminated and the cells were characterised by FACS-analysis after detaching the cells from the bottom of the tissue culture flask as described in Example 2. It was found that the relative amount of CD14⁺-cells decreases in these set ups, from 92 % to 42 % in set up "Mo" and from 45 % to 28 % in set up "Mo+Ly". Seemingly, the lymphocytes proliferate quicker than the monocytes;the relative amount of lymphocytes increased in set up "Mo" from 4,8 % to 69,8 % and in set up "Mo+Ly" from 23,5 % to 50,6 %. During the cultivation, CD14⁺/CD3⁺-cells effective as TAIC are formed in both cultures. It is important in this context that a significantly higher increase in CD14⁺/CD3⁺-cells is observed in the set up "Mo+Ly" with an amount of 32,0 ± 5,3 % as opposed to set up "Mo" with only 7,2 ± 3,2 %.

These results demonstrate that the purification of the cells for enrichment of monocytes to a relative amount of more than 90 % at the beginning of the cultivation has a negative influence on the generation of the immuno suppressive CD14⁺/CD3⁺-cells in the TAIC population, while the method described in Examples 1 and 2 leads to a significantly higher yield of CD14⁺/CD3⁺-cells.

### Example 12

### In vitro co-cultivation of human TAIC with allogeneic lymphocytes for generating regulatory T-cells

To examine whether TAIC induce the formation of regulatory T-cells, i.e. CD4⁺/CD25⁺-lymphocytes, in the recipient, several different *in vitro* cultures of TAIC with lymphocytes, were run and the formation of regulatory T-cells resulting therefrom was analyzed.

For this purpose, TAIC of donor A were directly or indirectly co-cultivated *in vitro* together with lymphocytes of a donor B. In the direct co-culture, the direct cell-to-cell-contact between the TAIC (of donor A) and the lymphocytes (of donor B) was made possible, while in the indirect co-culture a membrane ("cell culture insert", 0,4 µm pore size, Falcon, order no. 353090) allowed exchange of the medium, but inhibited the physical contact of the two cell populations. The direct or indirect co-culture is carried out for preferably 3 to 5 days, more preferably for 4 days under incubator conditions i.e. at 37°C and in a 5% CO₂ atmosphere.

After culturing, the respective numbers of regulatory T-cells (CD4⁺/CD25⁺) were determined in both set ups as well as in the controls, wherein TAIC or lymphocytes respectively were cultivated alone. Further, the number of CD3⁺/CD14⁺-cells, which represent the component of the TAIC-cell population having the most significant suppressor function in the mixed lymphocyte culture, was determined in the control set up, wherein the TAIC were cultured alone.

In all set ups, the surface antigens of the cells were determined per FACS analysis and the amount of the respective cell populations in the total amount of the cells was analysed.

Further, the relative expression of three new master genes specifically expressed by regulatory T-cells (Foxp3, CTLA-4 and Integrin α_{E}β₇) was determined by PCR in the respective cell population (see Table). Foxp3 is a specific transcription factor, which is viewed as a control gene for the development of regulatory T-cells, and which is specifically expressed by these cells [see Hori, S. et al., "Control of Regulatory T-cell Development by the Transcription Factor Foxp3", Science 299, 1057 - 1061 (2003)].

CTLA-4 is a further factor, which is used as a marker for the determination of the regulatory function of CD4⁺/CD25⁺-T-cells (see Khattri, R. et al., "An essential role for Scurfin in CD4+/CD25+ T regulatory cells", Nature Immunology, online publication, doi:10.1038/ni909 (2003); Shimizu, J. et al. "Stimulation of CD25+/CD4+ regulatory T cells through GITR breaks immunological self-tolerance", Nature Immunology, online publication, doi:10.1038/ni759 (2003); Cobbold, S.P. et al. "Regulatory T cells in the induction and maintenance of peripheral transplantation tolerance", Transpl. Int. 16(2), 66-75 (2003)].

Integrin α_{E}β₇ is an integrin, which binds to epithelial Catherine and which may be used as a marker for the most potent sub-population of regulatory CD25⁺-T-cells [see Lehmann, J. et al. "Expression of the integrin αEβ7 identifies unique subsets of CD25+ as well as CD25- regulatory T cells" PNAS 99(20), 13031-13036 (2002)].

The determination of Foxp3-, CTLA-4- and integrin α_{E}β₇-expession was carried out by means of quantitative PCR using GAPDH and β-actin, two "housekeeping genes", as controls; the values determined were, on the one hand, placed in relationship to each other, the value obtained for the CD14⁺/CD3⁻ cells being set at 1; on the other hand the absolute RNA amounts were indicated in µg per µg total RNA, in order to correlate the measured' suppression effected by the TAIC *in vitro* and the formation of the CD4⁺/CD25⁺ double positive cells with the expression rate of the respective genes. Standard methods were used for the quantitative PCR, which are known to the skilled person (see Lottspeich, F., Zorbas, H. "Bioanalytik", Spektrum Akademischer Verlag GmbH, Heidelberg-Berlin, 1998).

The Table shows that within the population of lymphocytes obtained from the direct co-culture, the percentage of double positive cells CD4⁺/CD25⁺ increases manifold to a value of 8,7 % in comparison to the amount of CD4⁺/CD25⁺ lymphocytes obtained from the indirect co-culture or control, which is nearly identical with 2,38 % and 2,65 % respectively.

The sub-population of CD4⁺/CD25⁺ cells expresses the highest relative amounts of mRNA from all tested mastergenes as compared to the expression in CD4⁺/CD25⁻ cells (see Table). While the expression of Foxp3 is increased by about a factor 10 (37 versus 3,75), CTLA-4 expression reaches an even higher maximum expression% (4699 versus 0,376). The increase expresssion of the third mastergene integrin increases CD4⁺/CD25⁺ cells during co-culturing is nearly as high as for CTLA-4 since the absolute amount of integrin α_{E}β₇ mRNA is raised to 1,4 × 10⁻⁹ in CD4⁺/CD25⁺ cells as compared to 3,4 × 10⁻¹² µg mRNA/µg total RNA in CD4⁺/CD25⁻ cells.

After indirect co-culture, the relative amount of Foxp3-mRNA in the CD4⁺/CD25⁺ sub-population amounts to only 10 and is nearly as low as the relative amount expressed by the lymphocytes of the control, which expresses a relative amount of Foxp3-mRNA of 15.

The relative amount of CTLA-4-mRNA expressed by the lymphocytes of the control is as low as 0,375 in the CD4⁺/CD25⁺-population and 0,1 in the CD4⁺/CD25⁻population.

**Table: Determination of the amounts of specific cell populations in the total amount of cells after direct and indirect co-culture by reference to their surface markers CD3, CD4, CD14, CD25, and determination of the relative expression and the absolute amounts of RNA of three genes (Foxp3, CTLA-4 and integrin α_{E}β₇) in these cell populations.**

| | Control | | | | direct co-culture | | indirect co-culture | |
|---|---|---|---|---|---|---|---|---|
| | TAIC | | lymphocytes | | lymphocytes | | lymphocytes | |
| | CD14⁺/ CD3⁺ | CD14⁺/ CD3⁻ | CD4⁺/ CD25⁺ | CD4⁺/ CD25⁻ | CD4⁺/ CD25⁺ | CD4⁺/ CD25⁻ | CD4⁺/ CD25⁺ | CD4⁺/ CD25⁻ |
| FACS [% positive cells] | 41 | 20 | 2,65 | 30,8 | 8,7 | 49 | 2,38 | 27,6 |
| relative Foxp3-expression [PCR] | 50 | 1 | 15 | 1,5 | 37 | 3,76 | 10 | 1,5 |
| absolute RNA-amount Foxp3 | 1,6×10⁻⁸ | 3,2×10⁻¹⁰ | 4,8×10⁻⁹ | 8×10⁻¹⁰ | 1,2×10⁻⁸ | 1,21×10⁻⁹ | 3,2×10⁻⁹ | 4,8×10⁻¹⁰ |
| relative CTLA4-expression [PCR] | 12500 | 1 | 0,375 | 0,1 | 4699 | 0,376 | | |
| absolute RNA-amount CTLA4 | 6.5×10⁻⁶ | 5,2×10⁻¹⁰ | 1,9×10⁻¹⁰ | 5,2×10⁻¹⁰ | 2,4×10⁻⁶ | 1,9×10⁻¹⁰ | | |
| absolute RNA-amount Integrin α_{E}β₇ | 3,4×10⁻⁹ | not detectable | 1,4×10⁻⁹ | 3,4×10⁻¹² | | | | |

The expression of CTLA-4 in the CD14⁺/CD3⁺ sub-population of the TAIC cells was similar to the expression of Foxp3 and was also significantly higher than in all other cell populations.

In this regard, it is noteworthy that the CTLA-4 expression (relative value of 12.500) was manifold stronger in the CD14⁺/CD3⁺-sub-population than the Foxp3-expression, which exhibited a relative value of 50.

No expression of the integrin α_{E}β₇ was detectable in the CD14⁺/CD3⁻ sub-population of the TAIC, while similar to the behavior of the expression of the other two analyzed genes, the expression of the integrin in the CD14⁺/CD3⁺-sub-population of the TAIC, measured as absolute value in µg RNA per µg total RNA, reached the highest value (3,4 × 10⁹ µg / µg total RNA).

The significantly increased expression of the three lymphocyte markers Foxp3, CTLA-4 and integrin α_{E}β₇ on the TAIC derived from monocytes as compared to normal lymphocytes is totally unexpected and has to date never been observed on monocytes or on cells derived from monocytes.

In conclusion, the results of this Example as shown above demonstrate the following: If one starts from the assumption that the level of Foxp3-, CTLA-4-, and integrin α_{E}β₇- expression correlates with the immunoregulatory properties of the respective cells, it was demonstrated here, that the suppressor activity of the CD3⁺/CD14⁺ sub-population of the TAIC is associated with a high expression of these three genes. This is even more surprising when it is considered that these markers have to date only been described for lymphocytes, but never in cells of monocytic origin.

It was further shown that the direct co-culture of TAIC with lymphocytes leads to a significantly higher amount of CD4⁺/CD25⁺ lymphocytes as compared to indirect co-cultivation and the cultivation of only lymphocytes. This supports the assumption that also *in vivo* (i.e. in a patient) regulatory T-cells are formed after administration of TAIC. These results are in conformity with the known immunoregulatory function of CD4⁺/CD25⁺ lymphocytes and with the fact, that the contents of Foxp3-, CTLA-4-, and integrin α_{E}β₇-mRNA in these cells are significantly higher than in indirect co-cultivated lymphocytes or in control lymphocytes.

### Example 13

### In vivo Induction of Transplant Tolerance by Lymphocytes co-cultured with TAIC in vitro

The results and conclusions presented in Example 12 were confirmed *in vivo* in an animal experiment. Different from the procedure described in Examples 3, 4, 5, 6 and 7, the animals in the selectedLEW->DA Inbred combinations were not injected with LEW-derived TAIC for induction of tolerance, but DA-lymphocytes from the recipient which, over a period of 5 days, were previously directly co-cultivated (LEW)-derived TAIC from the donor, or which were cultivated alone in medium as controls, were injected to the animals in this example.

Animals, which received autologous co-cultivated DA-lymphocytes prior to an allogeneic heart transplantation, developed donor specific tolerance, whereas control animals, which received naive non-cocultivated control DA-lymphocytes, acutely rejected the transplanted heart within 10 to 14 days.

These results indicate that the physical cell to cell interaction between donor-TAIC and recipient-lymphocytes induces the generation of regulatory T-cells, which, per se, are capable of modifying the syngeneic immune system of the recipients such, that potentially alloreactive T-cells are suppressed and the organ rejection is thereby prevented.

## Claims

1. A process for preparing a pharmaceutical preparation containing transplant acceptance inducing cells of monocytic origin, **characterised in that**
a) monocytes are isolated from blood;
b) the monocytes are multiplied in a suitable culture medium which contains the cellular growth factor M-CSF;
c) the monocytes are cultivated simultaneously with or following step b) in a culture medium containing γ-IFN; and
d) the transplant acceptance inducing cells formed in step c) are obtained by separating the cells from the culture medium; and
e) the cells obtained in step d) are formulated into a pharmaceutical preparation.

2. A process according to claim 1 **characterised in that** the monocytes are of human origin.

3. A process according to claims 1 or 2 **characterised in that** the monocytes are isolated from the blood in such a manner that next to the monocytes also lymphocytes are present in an amount of at least 10% by reference to the total cell number in the isolate.

4. A process according to claims 1 to 3, **characterised in that** the transplant acceptance inducing cells formed in step c) or obtained in step d) are selected by binding to the antibody produced by the hybridoma cell line DSM ACC2542 and the so selected cells are formulated into a pharmaceutical preparation.

5. A process according to claims 1 to 4, **characterised in that** the M-CSF concentration in the culture medium is 1 to 20 µg/l.

6. A process according to claims 1 to 5, **characterised in that**, subsequent to step b) the monocytes are cultivated for 24 to 72 hours in a culture medium containing γ-IFN, the cultivation in the presence of γ-IFN beginning 3 to 6 days after the beginning of cultivation step b).

7. A process according to claim 6, **characterised in that** the γ-IFN concentration in the culture medium is 0.1 to 20 ng/ml.

8. A process according to claims 1 to 7 **characterised in that** the total cultivation period in steps b) and c) is 4 to 8 days.

9. Pharmaceutical preparation for the suppression of transplant rejection reactions obtained by the process of claims 1 to 8.

10. The use of transplant acceptance inducing cells obtained by steps a) to c) of the process of claims 1 to 8 for *in vitro* generating and/or propagating regulatory T-lymphocytes.

11. The use according to claim 10, wherein the regulatory T-lymphocytes co-express the antigens CD4 and CD25 on their cell surface.

12. A process for the generation and/or propagation of regulatory T-lymphocytes, **characterised in that**
a) transplant acceptance inducing cells obtained by steps a) to c) of the process of claims 1 to 8 are co-cultivated with a T-lymphocyte preparation, and
b) the regulatory T-lymphocytes are optionally obtained from the culture medium.

13. A process according to claim 12, **characterised in that** the regulatory T-lymphocytes co-express the antigens CD4 and CD25 on their cell surface.

14. A process according to claims 12 or 13, **characterised in that** the regulatory T-lymphocytes are obtained from the culture medium by FACS sorting.

## Patentansprüche

1. Verfahren zur Herstellung einer pharmazeutischen Zusammensetzung, welche Transplantat-Akzeptanz induzierende Zellen monozytären Ursprungs enthält, **dadurch gekennzeichnet, dass** man
a) Monozyten aus Blut isoliert;
b) Monozyten in einem geeigneten Kulturmedium vermehrt, welches den zellulären Wachstumsfaktor M-CSF enthält;
c) die Monozyten gleichzeitig mit oder im Anschluss an Stufe b) in einem γ-IFN enthaltendem Medium kultiviert;
d) die in Stufe c) gebildeten Transplantat-Akzeptanz induzierenden Zellen gewinnt, indem man die Zellen von dem Kulturmedium abtrennt; und
e) die in Stufe c) erhaltenen Zellen zu einer pharmazeutischen Zusammensetzung formuliert.

2. Verfahren nach Anspruch 1, **dadurch gekennzeichnet, dass** die Monozyten humanen Ursprungs sind.

3. Verfahren nach den Ansprüchen 1 oder 2, **dadurch gekennzeichnet, dass** die Monozyten auf solche Weise aus dem Blut isoliert werden, dass neben den Monozyten auch Lymphozyten in einer Menge von mindestens 10 %, bezogen auf die Gesamtzahl der Zellen, in dem Isolat enthalten sind.

4. Verfahren nach den Ansprüchen 1 bis 3, **dadurch gekennzeichnet, dass** die in Stufe c) gebildeten oder in Stufe d) erhaltenen Transplantat-Akzeptanz induzierenden Zellen durch Bindung an den Antikörper selektiert werden, welcher von der Hybridomzell-Linie DSM ACC2542 gebildet wird, und die so selektierten Zellen zu einer pharmazeutischen Zusammensetzung formuliert werden.

5. Verfahren nach den Ansprüchen 1 bis 4, **dadurch gekennzeichnet, dass** die M-CSF-Konzentration in dem Kulturmedium 1 bis 20 µg/l beträgt.

6. Verfahren nach den Ansprüchen 1 bis 5, **dadurch gekennzeichnet, dass** im Anschluss an Stufe b), die Monozyten 24 bis 72 Stunden lang in einem Kulturmedium kultiviert werden, welches γ-IFN enthält, wobei die Kultivierung in Gegenwart von γ-IFN 3 bis 6 Tage nach Beginn der Kultivierungsstufe b) beginnt.

7. Verfahren nach Anspruch 6, **dadurch gekennzeichnet, dass** die γ-IFN-Konzentration in dem Kulturmedium 0,1 bis 20 ng/ml beträgt.

8. Verfahren nach den Ansprüchen 1 bis 7, **dadurch gekennzeichnet, dass** die Gesamtdauer der Kultivierung in den Stufen b) und c) 4 bis 8 Tage beträgt.

9. Pharmazeutische Zusammensetzung zur Suppression von Transplantat-Abstossungsreaktionen, hergestellt nach dem Verfahren der Ansprüche 1 bis 8.

10. Verwendung von Transplantat-Akzeptanz induzierenden Zellen, die anhand der Schritte a) bis c) des Verfahrens nach den Ansprüchen 1 bis 8 erhalten wurden, zur in vitro-Gewinnung und/oder Vermehrung regulatorischer T-Lymphozyten.

11. Verwendung nach Anspruch 10, **dadurch gekennzeichnet, dass** die regulatorischen T-Lymphozyten die Antigene CD4 und CD25 auf ihrer Zelloberfläche co-exprimieren.

12. Verfahren zum Generieren und/oder Propagieren regulatorischer T-Lymphozyten, **dadurch gekennzeichnet, dass** man
a) Transplantat-Akzeptanz induzierende Zellen, hergestellt gemäß den Stufen a) bis c) des Verfahrens nach den Ansprüchen 1 bis 8 mit einer T-Lymphozytenzubereitung cokultiviert, und
b) die regulatorischen T-Lymphozyten gegebenenfalls aus dem Kulturmedium gewinnt.

13. Verfahren nach Anspruch 12, **dadurch gekennzeichnet, dass** die regulatorischen T-Lymphozyten die Antigene CD4 und CD25 auf ihrer Zelloberfläche co-exprimieren.

14. Verfahren nach den Ansprüchen 12 oder 13, **dadurch gekennzeichnet, dass** man die regulatorischen T-Lymphozyten aus dem Kulturmedium durch FACS-Sortieren gewinnt.

## Revendications

1. Procédé pour préparer une préparation pharmaceutique contenant des cellules d'origine monocytaire induisant l'acceptation d'un transplant, **caractérisé en ce que**
a) des monocytes sont isolés à partir du sang ;
b) les monocytes sont multipliés dans un milieu de culture approprié qui contient le facteur de croissance cellulaire M-CSF ;
c) les monocytes sont cultivés simultanément à ou après l'étape b) dans un milieu de culture contenant de l'IFN-γ ;
d) les cellules induisant l'acceptation du transplant formées dans l'étape c) sont obtenues en séparant les cellules du milieu de culture ; et
e) les cellules obtenues dans l'étape d) sont formulées en une préparation pharmaceutique.

2. Procédé selon la revendication 1, **caractérisé en ce que** les monocytes sont d'origine humaine.

3. Procédé selon la revendication 1 ou 2, **caractérisé en ce que** les monocytes sont isolés à partir du sang de telle manière qu'en plus des monocytes, des lymphocytes sont également présents en une quantité d'au moins 10 % par rapport au nombre total de cellules dans l'isolat.

4. Procédé selon la revendication 1 ou 3, **caractérisé en ce que** les cellules induisant l'acceptation du transplant formées dans l'étape c) ou obtenues dans l'étape d) sont sélectionnées par liaison à l'anticorps produit par la lignée cellulaire d'hybridome DSM ACC2542, et les cellules ainsi sélectionnées sont formulées en une préparation pharmaceutique.

5. Procédé selon les revendications 1 à 4, **caractérisé en ce que** la concentration de M-CSF dans le milieu de culture est de 1 à 20 µg/l.

6. Procédé selon les revendications 1 à 5, **caractérisé en ce que**, après l'étape b), les monocytes sont cultivés pendant 24 à 72 heures dans un milieu de culture contenant de I'IFN-γ, la culture en présence d'IFN-γ commençant 3 à 6 jours après le début de l'étape de culture b).

7. Procédé selon la revendication 6, **caractérisé en ce que** la concentration d'IFN-γ dans le milieu de culture est de 0,1 à 20 ng/ml.

8. Procédé selon les revendications 1 à 7, **caractérisé en ce que** la durée totale de culture dans les étapes b) et c) est de 4 à 8 jours.

9. Préparation pharmaceutique pour la suppression des réactions de rejet de transplant obtenue par le procédé des revendications 1 à 8.

10. Utilisation de cellules induisant l'acceptation d'un transplant obtenues par les étapes a) à c) du procédé selon les revendications 1 à 8 pour la production et/ou la multiplication *in vitro* de lymphocytes T régulateurs.

11. Utilisation selon la revendication 10, dans laquelle les lymphocytes T régulateurs co-expriment les antigènes CD4 et CD25 sur leur surface cellulaire.

12. Procédé pour la production et/ou la multiplication *in vitro* de lymphocytes T régulateurs, **caractérisé en ce que**
a) des cellules induisant l'acceptation du transplant obtenues par les étapes a) à c) du procédé selon les revendications 1 à 8 sont co-cultivées avec une préparation de lymphocytes T, et
b) en option, les lymphocytes T régulateurs sont obtenus à partir du milieu de culture.

13. Procédé selon la revendication 12, **caractérisé en ce que** les lymphocytes T régulateurs co-expriment les antigènes CD4 et CD25 sur leur surface cellulaire.

14. Procédé selon la revendication 12 ou 13, **caractérisé en ce que** les lymphocytes T régulateurs sont obtenus à partir du milieu de culture par tri par FACS.
